(19) Europäisches Patentamt
European Patent Office
Office européen des brevets

(11) EP 3 050 886 A1

(12) EUROPEAN PATENT APPLICATION

(43) Date of publication:
03.08.2016 Bulletin 2016/31

(21) Application number: 15153463.3

(22) Date of filing: 02.02.2015

(51) Int Cl.:
*C07D 493/10* (2006.01) *A61K 31/35* (2006.01)
*A61K 31/365* (2006.01) *C09B 11/06* (2006.01)
*C09K 11/06* (2006.01) *C09K 11/59* (2006.01)
*C09K 11/66* (2006.01) *C12N 1/00* (2006.01)
*C12Q 1/02* (2006.01) *G01N 33/48* (2006.01)
*G01N 33/50* (2006.01) *G01N 21/64* (2006.01)
*G01N 33/52* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Bürkert Werke GmbH**
**74653 Ingelfingen (DE)**

(72) Inventors:
- **Lelant, Célia**
**59700 Marcq-en-Baroeul (FR)**
- **Bonnet, Dominique**
**67118 Geisposlsheim (FR)**
- **Galzi, Jean-Luc**
**67500 Weitbruch (FR)**

(74) Representative: **Prinz & Partner mbB**
**Patent- und Rechtsanwälte**
**Rundfunkplatz 2**
**80335 München (DE)**

(54) **Fluorescent dyes and dye precursors**

(57) A xanthene compound is used for discriminating living from non-living unicellular and multicellular organisms. The xanthene compound is represented by the following formula (I)

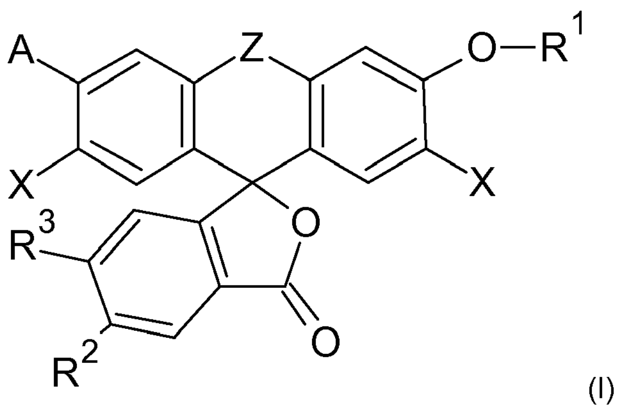

(I)

wherein
each X is, independently from each other, one of H, F, Cl, Br, I, CN;
Z is one of O, Si, Ge or Sn;
$R^1$ is hydrogen, -(C=O)-$R^4$ or -$CH_2$-O-(C=O)-$R^4$, wherein $R^4$ is substituted or unsubstituted alkyl having 1 to 18 carbon atoms, substituted or unsubstituted alkenyl having 2 to 18 carbon atoms, or substituted or unsubstituted aralkyl having at least 7 carbon atoms, or wherein $R^1$ is a residue derived from an amino acid, peptide, nucleotide or oligonucleotide, monosaccharide or polysaccharide;

A is -$NH_2$, -O$R^1$ or -N$R^5$(C=O)-$R^4$ wherein $R^4$ independently has the meaning as defined above and $R^5$ is H, substituted or unsubstituted alkyl having 1 to 18 carbon atoms, substituted or unsubstituted alkenyl having 2 to 18 carbon atoms, or substituted or unsubstituted aralkyl having at least 7 carbon atoms;

$R^2$ and $R^3$ are, independently from each other, H or -(C=O)-N$R^6R^7$, wherein at least one of $R^2$ and $R^3$ is not H, and

EP 3 050 886 A1

wherein $R^6$ and $R^7$ are selected from H, substituted or unsubstituted alkyl having 1 to 18 carbon atoms, substituted or unsubstituted alkenyl having 2 to 18 carbon atoms, or substituted or unsubstituted aralkyl having at least 7 carbon atoms.

# Fig. 1

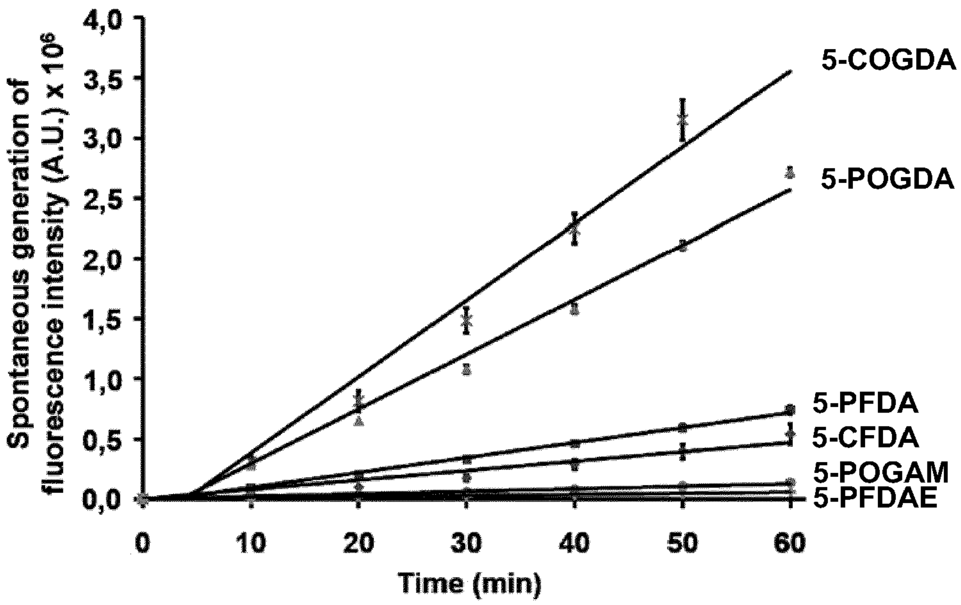

## Description

FIELD OF THE INVENTION

The invention relates to fluorescent dyes and dye precursors, and in particular to cell permeating xanthene compounds, as well as the use of these compounds for discriminating living from non-living unicellular and multi-cellular organisms.

BACKGROUND OF THE INVENTION

Fluorescent dyes generally bear charged chemical groups or functions that hinder their penetration into cells. Chemical groups such as ester groups can be used to mask the charge and/or mask the fluorescence properties in a reversible manner. Such a chemical group is called "protective group" or "blocking group" in the following, and their grafting onto dyes leads to blocked dye precursors or pro-dyes.

In the process of discriminating living cells from non living cells, the protective groups are selected for their capacity to both permit dye penetration into cells by passive diffusion, and at the same time mask the fluorescent properties of the dye. The living cell, in contrast to the non-living (dead) cell, contains enzymes that can unblock the dye precursor by cleaving the protective group. This cleavage has two consequences: the dyes become sequestered in the cell resulting in an accumulation of the dyes, and their fluorescence properties are relieved so that the dyes become detectable.

US-A-4318846 describes symmetrically substituted diether fluoresceins having at least an anionic group and a linking functionality. At least some of the substituents on the aromatic rings may be halogen including fluorine. The compounds are used in fluorescent immunoassays.

WO 1997/039064 A1 discloses the preparation of xanthene derivatives including fluoresceins and rhodols that are directly substituted on one or more aromatic carbon atoms by fluorine. These fluorinated fluorescent dyes are useful as detectable tracers and for preparing conjugates of organic and inorganic substances.

The article of Yuichiro KOIDE et al., "Evolution of group 14 rhodamines as platforms for near-infrared fluorescence probes utilizing photoinduced electron transfer", ACS Chem. Biol.6, 600-608 (2011), describes the preparation of pyronine and rhodamine analogs in which the oxygen atom at position 10 is replaced by Si, Ge or Sn to provide a large bathochromic shift to the original rhodamines. These molecules retain the advantages of the original rhodamines, including high quantum efficiencies in aqueous media, tolerance to photobleaching and high water solubility. They are thus suitable for intravital labeling of living cells with high signal-to-noise ratio.

Carboxyfluorescein diacetate (CDFA), as a dye precursor for intravital labeling of living cells using host cell esterases to unblock hydroxyl groups, is disclosed in the following publications:

a) T.H. Chrzanowski et al., "Applicability of the fluorescein diacetate method of detecting active bacteria in fresh water", Microb Ecol (1984) 10:179-185; and

b) Thomas A. Robertson, et al., "Fluorescein Derivatives in Intravital Fluorescence Imaging", Cells 2013, 2(3), 591-606.

The article of L. D. Lavis et al., "Synthesis and utility of fluorogenic acetoxymethyl ethers", Chem Sci., 2011, 2, 521-530 discloses that acetoxy-methyl esters of fluoresceine (4a) and Oregon green (4b) are suitable profluorophores for intravital labeling of cells:

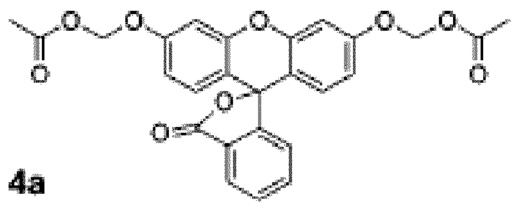

4a

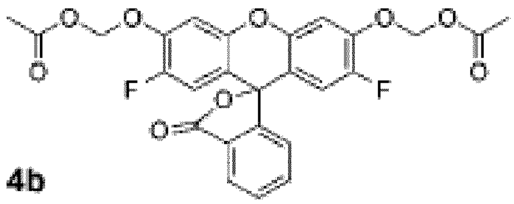

4b

In an article of D. Hoefel et al., "A comparative study of carboxyfluorescein diacetate and carboxyfluorescein diacetate succinimidyl ester as indicators of bacterial activity", Journal of Microbiological Methods 52 (2003) 379-388, it was found that carboxyfluorescein diacetate (CFDA) was successful in detecting active bacteria, by qualitative and quantitative analysis of exponential phase cultures, mixtures of active and inactive cells and bacteria from environmental waters whereas carboxyfluorescein diacetate succinimidyl ester (CFDA/SE) was not. CFDA/SE labelled inactive cells with intensities equal to that of the active population and could not even discriminate between bacteria in exponential phase growth and a fixed cell preparation.

The article of P. Breeuwer et al., "Characterization of uptake and hydrolysis of fluorescein diacetate and carboxyfluorescein diacetate by intracellular esterases in Saccharomyces cerevisiae, which result in accumulation of fluorescent product", Appl Environ Microbiol. Apr 1995; 61(4): 1614-1619, shows that not only bacteria, but also yeast cells can be labeled with fluorescein and carboxyfluoresceine derivatives.

The article by Vince Boyd et al., "Limitations in the Use of Fluorescein Diacetate/Propidium Iodide (FDA/PI) and Cell Permeable Nucleic Acid Stains for Viability Measurements of Isolated Islets of Langerhans", Curr Trends Biotechnol Pharm . 2008 March ; 2(2): 66-84, shows that also mammalian cells can be stained using fluorescein diacetate.

FR 2 955 121 A claims a culture medium for microorganisms, comprising a nutrient medium in which are homogeneously dissolved at least one fluorogenic substrate and a compound for masking residual fluorescence associated with non-specific activation of said fluorogenic substrate. CFDA is used as the fluorogenic substrate in combination with a contrast agent to reveal living bacteria.

WO2006003696 (A1) entitled "Method of Determining Viable Cell Count and Apparatus Therefor" focuses on the use of CFDA for the enumeration of viable cells in a sample, including microbes. A specimen is stained with the use of carboxyfluorescein diacetate and trypan blue, and irradiated with carboxyfluorescein diacetate excitation light. The fluorescence emitted by the specimen is collected, captured as an image, and converted to an electrical signal to thereby determine a viable cell count.

JP2004187534 (A) entitled "Method for Evaluating Physiological Activity of Microorganism or Cell and Kit Therefor" relates to a method of analyzing the physiological activity of cells based on a fixation step, a revelation step using CFDA and a washing step. In particular, the method comprises a process for collecting the microorganism or the cell by adhesion or transfer with an adhesive sheet, a process for dyeing the microorganism or the cell with a solution containing carboxyfluorescein diacetate, and a process for cleaning the dyed microorganism or cell with a cleaning liquid.

US2008220439 A1 relates to a microorganism detecting kit including a self-adhesive layer containing the following compounds incorporated therein: 4',6-diamidino-2-phenylindole dihydrochloride for staining living and dead cells, propidium iodide 3 for staining dead cells, 6-carboxyfluorescein diacetate for staining living cells and 4-methylumbelliferyl-beta-D-galactoside reacting with a substance derived from particular microorganisms, a support and a grip portion. Microorganisms deposited to a specimen are deposited to the self-adhesive layer, and then the compounds react with the microorganisms and are bonded individually to the microorganisms to color them. The microorganism detecting kit is used to confirm of the presence of living and dead cells, dead cells and living cells by counting the colored cells in an adding manner.

There is still a need for improved fluorescent dye precursors ("pro-fluorophores") which can be used for discriminating living from non-living organisms in biological or industrial samples.

SUMMARY OF THE INVENTION

In one aspect, the invention is a xanthene derivative including blocked fluorescein and rhodol derivatives having an improved permeability through the cell membrane of bacteria and other living organisms. The xanthene derivatives of the present invention are useful pro-fluorophores for cell viability tests such as in drinking water or other samples.

In a further aspect, the invention is directed to the fluorophores obtained by unblocking the xanthenes pro-fluorophores of the first aspect of the invention.

The inventors found that carboxyfluorescein diacetate (CFDA) still has a free carboxylic acid group in 5- or 6-position at the bottom ring:

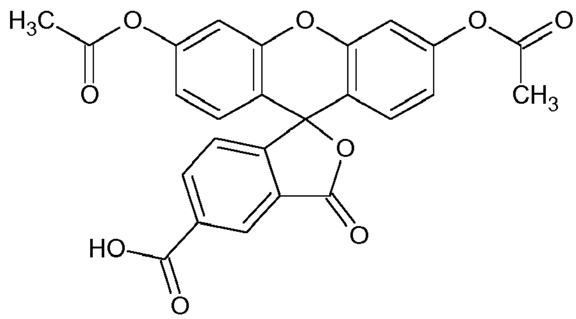

At physiological pH, this carboxylic acid group is mostly ionized ($-COO^-$) which makes it less prone to diffusion through biological membranes that are made up of hydrophobic and apolar lipid chains. Accordingly, the free acid group on CFDA is likely to significantly reduce the fraction of reagent entering the cells.

The inventors therefore contemplate that blocking the free carboxylic acid group in addition to the hydroxyl groups would improve the efficacy of the xanthene dye precursors.

Accordingly, the present invention provides a compound represented by the following formula (I)

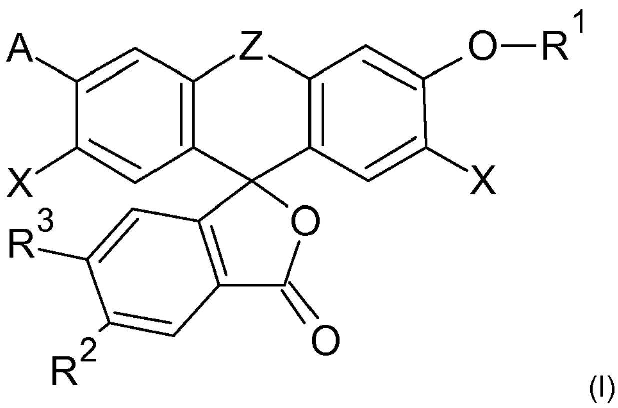

(I)

wherein
each X is, independently from each other, one of H, F, Cl, Br, I, CN;
Z is one of O, Si, Ge and Sn;
$R^1$ is hydrogen, $-(C=O)-R^4$ or $-CH_2-O-(C=O)-R^4$, wherein $R^4$ is substituted or unsubstituted alkyl having 1 to 18 carbon atoms, substituted or unsubstituted alkenyl having 2 to 18 carbon atoms, or substituted or unsubstituted aralkyl having at least 7 carbon atoms, or wherein $R^1$ is a residue derived from an amino acid, peptide, nucleotide or oligonucleotide, monosaccaride or polysaccharide;
A is $-NH_2$, $-OR^1$ or $-NR^5(C=O)-R^4$ wherein $R^4$ independently has the meaning as defined above and $R^5$ is H, substituted or unsubstituted alkyl having 1 to 18 carbon atoms, substituted or unsubstituted alkenyl having 2 to 18 carbon atoms, or substituted or unsubstituted aralkyl having at least 7 carbon atoms;
$R^2$ and $R^3$ are, independently from each other, hydrogen or $-(C=O)-NR^6R^7$, wherein at least one of $R^2$ and $R^3$ is not hydrogen, and wherein $R^6$ and $R^7$ are selected from hydrogen, substituted or unsubstituted alkyl having 1 to 18 carbon atoms, substituted or unsubstituted alkenyl having 2 to 18 carbon atoms, or substituted or unsubstituted aralkyl having at least 7 carbon atoms.

Optional substituents are preferably selected from the group consisting of F, Cl, Br, I, CN, hydroxyl, ether, amino, carboxylic acid, carboxylic acid esters of an aliphatic alcohol or phenol.

In a specific embodiment, the compound is represented by the following formula (II):

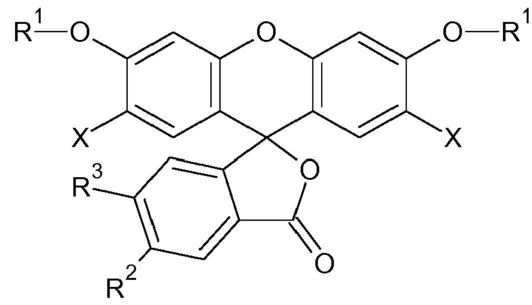

(II)

wherein X, $R^2$ and $R^3$ each have the meaning as defined above, each $R^1$ are the same or different from each other and are hydrogen, $-(C=O)-R^4$ or $-CH_2-O-(C=O)-R^4$, wherein $R^4$ independently is substituted or unsubstituted alkyl having 1 to 18 carbon atoms, substituted or unsubstituted alkenyl having 2 to 18 carbon atoms, or substituted or unsubstituted aralkyl having at least 7 carbon atoms.

More preferably, X is H or F, and $R^4$ independently is alkyl having 1 to 8 carbon atoms, more preferably methyl or ethyl.

$R^2$ or $R^3$ preferably is $-(C=O)-NH-R^7$ wherein $R^7$ is alkyl having 1 to 8 carbon atoms, more preferably alkyl having 1 to 4 carbon atoms.

In a more preferred embodiment, X in formula (II) is H, both $R^1$ are either $-(C=O)-R^4$ or $-CH_2-O-(C=O)-R^4$, $R^4$ is methyl, and one of $R^2$ and $R^3$ is $-(C=O)-NH-R^7$, wherein $R^7$ is selected from methyl, ethy, propyl, isopropy, butyl, isobutyl, more preferably propyl.

In a further preferred embodiment, X in formula (II) is F both $R^1$ are either $-(C=O)-R^4$ or $-CH_2-O-(C=O)-R^4$, $R^4$ is methyl, and one of $R^2$ and $R^3$ is $-(C=O)-NH-R^7$ wherein $R^7$ is selected from methyl, ethy, propyl, isopropy, butyl, isobutyl, more preferably propyl.

In another specific embodiment, the compound is represented by the following formula (III):

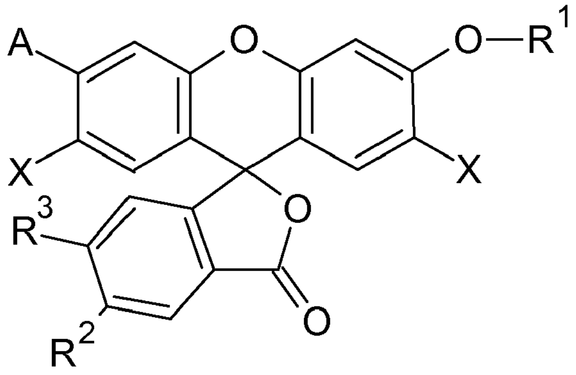

(III)

wherein X, $R^2$ and $R^3$ have the meaning as defined above, $R^1$ is $-(C=O)-R^4$ or $-CH_2-O-(C=O)-R^4$, and A is $-NH_2$, or $-NR^5(C=O)-R^4$, wherein each $R^4$ independently is substituted or unsubstituted alkyl having 1 to 18 carbon atoms, substituted or unsubstituted alkenyl having 2 to 18 carbon atoms, or substituted or unsubstituted aralkyl having at least 7 carbon atoms, and wherein $R^5$ is H, substituted or unsubstituted alkyl having 1 to 18 carbon atoms, substituted or unsubstituted alkenyl having 2 to 18 carbon atoms, or substituted or unsubstituted aralkyl having at least 7 carbon atoms.

More preferably, X is H or F, A is $-NR^5(C=O)-R^4$, $R^4$ independently is alkyl having 1 to 4 carbon atoms, more preferably methyl or ethyl, and $R^5$ is H or alkyl having 1 to 4 carbon atoms, more preferably hydrogen, methyl or ethyl.

$R^2$ or $R^3$ preferably is $-(C=O)-NH-R^7$ wherein $R^7$ is alkyl having 1 to 8 carbon atoms, more preferably alkyl having 1 to 4 carbon atoms.

In a more preferred embodiment, X in formula (III) is H, $R^1$ is either $-(C=O)-R^4$ or $-CH_2-O-(C=O)-R^4$, $R^4$ is methyl, $R^5$ is H, and one of $R^2$ and $R^3$ is $-(C=O)-NH-R^8$ wherein $R^8$ is selected from methyl, ethy, propyl, isopropy, butyl, isobutyl, more preferably propyl.

In a further preferred embodiment, X in formula (II) is F, $R^1$ is either $-(C=O)-R^4$ or $-CH_2-O-(C=O)-R^4$, $R^4$ is methyl, $R^5$ is H, and one of $R^2$ and $R^3$ is $-(C=O)-NH-R^8$ wherein $R^8$ is selected from methyl, ethy, propyl, isopropy, butyl, isobutyl, more preferably propyl.

In a further aspect, the compounds according to the invention are used for staining a biological sample.

Preferably, the biological sample comprises a living cell, and more preferably the living cell is a unicellular or multicellular organism.

In a preferred embodiment, the sample is incubated with the compound according to the invention for a time sufficient for the compound to yield a detectable fluorescence emission, and more preferably, the fluorescence emission is evaluated to discriminate living from non-living cells. More specifically, the above compounds are used in a method to discriminate living cells from non-living cells by incubating a sample containing the cells with a compound according the invention, and determining the fluorescence emitted from the compounds after cleavage of the protective groups -(C=O)-$R^4$ by enzymes present in the living cells.

In a preferred method, the compounds are used to determine the presence of bacteria such as *E. coli* in food samples and/or drinking water.

The above methods preferably comprise the step of subjecting the incubated biological sample to a cytometric assay or an immunological assay.

In a further aspect, the invention provides a culture medium for microorganisms, comprising a compound according to the invention dissolved in the culture medium, and optionally a nutrient medium. Suitable nutrient media for microorganisms are known to the person skilled in the art, as shown for example in FR 2 955 121 A.

The compounds according to the invention may also be used in combination with other fluorescent dyes, contrast media or other material such as propidium iodide and/or 4-methylumbelliferyl-beta-D-galactoside, as may be required by any specific assay.

In a preferred embodiment, the stained biological sample is treated with a contrast agent. More preferably, the contrast agent is selected from the group consisting of Brilliant Blue R, Trypan Blue and Bromophenol Blue.

## BRIEF DESCRIPTION OF THE DRAWINGS

The invention is now described in more detail with reference to specific embodiments and the drawings which are understood as non-limiting examples. In the drawings:

- Figure 1 is a graph showing the short-term stability of fluorescein derivatives according to the present invention;

- Figure 2 is a graph showing the long-term stability of fluorescein derivatives according to the present invention;

- Figure 3 shows a series of photomicrographs illustrating the labeling of different bacteria using pro-fluorophores according to the present invention, wherein (a) shows viable or dead waterborne *Enterobacter cloacae,* (b) shows *Chryseobacterium indologenes,* (c) shows *Pseudomonas aeruginosa,* and (d) shows *Bacillus subtilis;*

- Figure 4 shows a series of photomicrographs illustrating the labeling of viable *E. coli* using xanthenes derivatives of the present invention;

- Figure 5 shows a graph illustrating the relative rate of fluorescence intensity in viable or dead *E. coli* using various pro-fluorophores of the present invention; and

- Figure 6 shows a graph illustrating the effect of incubation time on the fluorescence intensity of stained viable *E. coli.*

## DESCRIPTION OF PREFERRED EMBODIMENTS

### Synthesis of fluorophores and pro-fluorophores derived from fluorescein and Oregon Green™

Techniques currently used to evaluate bacterial viability are based on the use of fluorogenic substrates. One of these substrates well described and adapted for this application is 5-carboxyfluorescein diacetate (5-CFDA). However, 5-CFDA has a carboxylic acid functional group bearing a negative charge according to the pH (~7) of the solution used to stain bacteria, which reduces the uptake of the pro-fluorophore in the bacteria.

According to the invention, new fluorogenic esterase substrates are used to improve the detection of viable *E. coli* (gram-negative bacteria) by improving intracellular delivery using different uncharged functional groups. Fluorine and acetoxymethyl (AM) substitution are also proposed in order to modify the hydrolysis speed of the pro-fluorophore according to the invention. After pro-fluorophore hydrolysis, the fluorophores are retained more efficiently inside the bacterial cell.

Reagents were obtained from commercial sources and used without further purification. Particular fluorophore based reagents are:

5-carboxyfluorescein succinimidyl ester (from Life Technologies);

Oregon Green® 488 carboxylic acid (from Life Technologies);

fluorescein isothiocyanate isomer I (from Sigma Aldrich); and

5,6-carboxyfluorescein (from Kodak).

Semi preparative reverse-phase high performance liquid chromatography (RP-HPLC) separations were performed on a SunFire™ C18 OBD™ Prep column (5 μm, 19 mm x 150 mm) from Waters™ using a linear gradient of solvent B in solvent A of 5% to 100% for 25 min, wherein solvent A was water with 0.1% trifluoroacetic acid (TFA) and solvent B was acetonitrile with 0.1% TFA. The flow rate was 20 ml/min; detection was at 220 nm and 254 nm.

Analytical reverse-phase high performance liquid chromatography (RP-HPLC) separations were performed on a Ascentis® Express C18 column (2.7 μm, 4.6 mm x 75 mm) from Supelco™ using a linear gradient of 5% to 100% of solvent B in solvent A for 7.5 min. The flow rate was 1.6 ml/min, and detection was at 220 nm and 254 nm.

Purified final compounds eluted as single and symmetrical peaks at retention times ($t_R$) given below. Their identity was determined by high resolution mass spectroscopy (HRMS). Intermediates were analyzed by liquid chromatography mass spectroscopy (LCMS) using a multi-mode MM-ES+APCI source.

Example 1 (Not Inventive)

**1-(3',6'-dihydroxy-3-oxo-3H-spiro[isobenzofuran-1,9'-xanthen]-5-yl)-3-propylthiourea (5-PFT)**

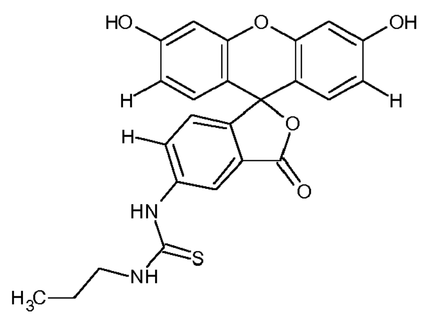

*N,N*-diisopropylethylamine (DIEA) (25 μl, 154 μmol) and propylamine (4.2 μl, 51 μmol) were added to a solution of fluorescein-5-isothiocyanate (10 mg, 25 μmol) in anhydrous DMSO (5 ml) The reaction mixture was stirred overnight in the dark, at room temperature and freeze-dried to yield the compound as an orange oil. The crude product was used in the next step without further purification. $t_R$ = 4.10 min, RP-HPLC purity: >90%, MS (ESI) m/z: 449.0 $(M+H)^+$

Example 2

**2',7'-difluoro-3',6'-dihydroxy-3-oxo-N-propyl-3H-spiro[isobenzofuran-1,9'-xanthene]-5-carboxamide (5-POG)**

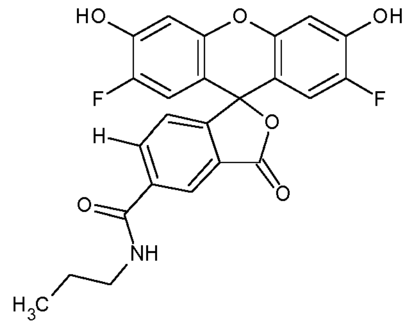

N,N-diisopropylethylamine (DIEA) (7.8 μl, 47 μmol) and propylamine (2 μl, 23.5 μmol) were added to a solution of Oregon Green® 488 carboxylic acid succinimidyl ester, isomer 5 (4 mg, 7.8 μmol) in anhydrous DMSO (2.5 ml). The reaction mixture was stirred in the dark overnight at room temperature, and freeze-dried to yield the compound as an orange oil. The crude product was used in the next step without further purification. $^1$H NMR (DMSO, 500 MHz): δ 10.78 (bs, 1 H), 8.82 (t, J = 5.6 Hz, 1 H), 8.47 (s, 1 H), 8.26 (dd, J = 8.1 Hz, J = 1.6 HZ, 1H), 7.39 (d, J = 8.2 Hz, 1H), 6.89 (d, 7.6 Hz, 2H), 6.58 (d, J = 11.3 Hz, 2H), 1.60-1.55 (m, 2H), 0.92 (t, J = 7.4 Hz, 3H); $t_R$ = 4.03 min, RP-HPLC purity: >90%, MS (ESI) m/z: 454.0 $(M+H)^+$

Example 3

**3',6'-dihydroxy-3-oxo-N-propyl-3H-spiro[isobenzofuran-1,9'-xanthene]-5-carboxamide (5-PFA)**

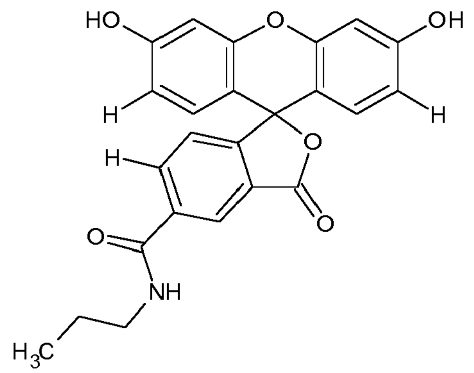

DIEA (10 μl, 64 μmol) and propylamine (2.6 μl, 31.7 μmol) were added to a solution of 5-carboxyfluorescein succinimidyl ester (5 mg, 10.5 μmol) in anhydrous DMSO (2.5 ml). The reaction mixture was stirred in the dark overnight at room temperature, and freeze-dried to yield the compound as an orange oil. The crude product was used in the next step without further purification. $^1$H NMR (DMSO, 500 MHz): δ 10.16 (bs, 1H), 8.81 (t, J = 5.4 Hz, 1H), 8.46 (d, J = 0.5 Hz, 1 H), 8.24 (dd, J = 8.1 Hz, J = 1.6 Hz, 1 H), 7.37 (d, J = 8 Hz, 1 H), 6.59-6.53 (m, 4H), 3.29-3.25 (m, 2H), 1.59-1.55 (m, 2H), 0.92 (t, J = 7.4 Hs, 3H); $^{13}$C NMR (DMSO, 125 Mhz): δ 168.2, 164.5, 159.6, 154.5, 151.8, 136.4, 134.7, 127.2, 126.4, 124.2, 123.2, 112.7, 109.1, 102.3, 41.2, 22.3, 11.5. $t_R$ = 3.79 min, RP-HPLC purity: >90%, %, MS (ESI) m/z: 418.0 $(M+H)^+$.

Example 4 (Not Inventive)

**Propyl-3',6'-dihydroxy-3-oxo-3H-spiro[isobenzofuran-1,9'-xanthene]-5-carboxylate & propyl-3',6'-dihydroxy-3-oxo-3H-spiro[isobenzofuran-1,9'-xanthene]-6-carboxylate (5,6-PFE)**

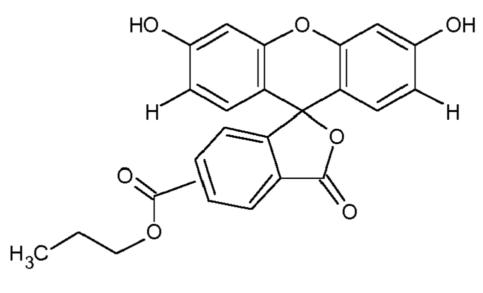

DIEA (33 μl, 0.2 mmol) and 1-bromopropane (12 μl, 0.13 mmol) were added to a solution of 5,6-carboxyfluorescein (25 mg, 66 μmol) in anhydrous DMSO (1 ml). The reaction mixture was stirred at 35 °C in the dark for 48 h and purified by reverse-phase HPLC to yield isomer 5 (10.7 mg, 38%) and isomer 6 (9.6 mg, 35%) as orange solids. For isomer 5: $t_R$ = 4.96 min, RP-HPLC purity: >95%, HR-MS for $C_{24}H_{18}O_7$: calculated 418.1053, found 418.1033.

General procedure for acetylation

Pyridine (0.52 mmol, 60 equiv.) and acetic anhydride (0.63 mmol, 50 equiv.) were added to a solution of a 3',6'-dihydroxy-xanthene compound (10 μmol, 1 equiv.) in a mixture of anhydrous $CH_2Cl_2$/DMF (2.4 ml, 5/1). The reaction mixture was stirred in the dark at room temperature for 4 h. All compounds were isolated by reverse-phase HPLC to give the desired product (18-83% yield).

Example 5 (Not Inventive)

**3-oxo-5-(3-propylthioureido)-3H-spiro[isobenzofuran-1,0'-xanthene]-3',6'-diyl diacetate (5-PFDAT)**

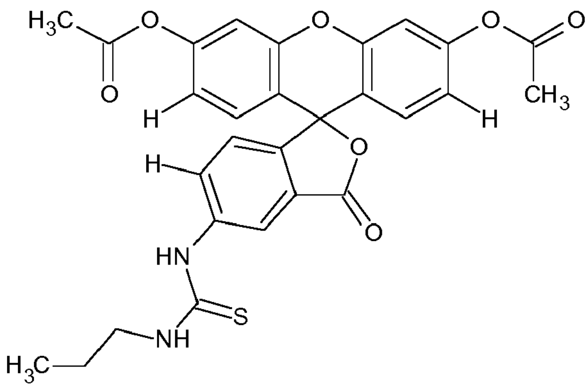

Acetylation of the compound of Example 1 resulted in a yellow solid; yield 2.4 mg (18% over 2 steps); $t_R$ = 5.37 min, RP-HPLC purity: ≥87%, HR-MS for $C_{28}H_{24}N_2O_7S$: calculated 532.1304, found 532.1310

Example 6

**2',7'-difluoro-3-oxo-5-(propylcarbamoyl)-3H-spiro[isobenzofuran-1,9'-xanthene]-3',6'-diyl diacetate (5-POGDA)**

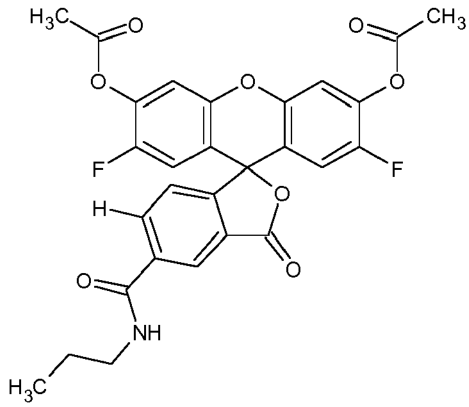

Acetylation of the compound of Example 2 resulted in a yellow solid; yield 4.5 mg (76% over 2 steps); $t_R$ = 5.51 min, RP-HPLC purity: ≥95%, HR-MS for $C_{28}H_{21}F_2NO_8$: calculated 537.1235, found 537.1236.

Example 7

**3-oxo-5-(propylcarbamoyl)-3H-spiro[isobenzofuran-1,9'-xanthene]-3',6'-diyl diacetate (5-PFDA)**

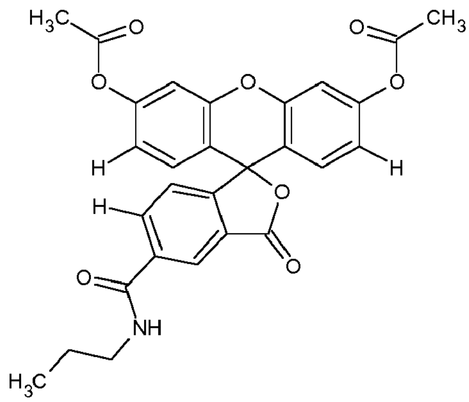

Acetylation of the compound of Example 3 resulted in a yellow solid; yield 3.2 mg (60% over 2 steps); $t_R$ = 5.21 min, RP-HPLC purity: ≥89%, HR-MS for $C_{28}H_{23}N_1O_8$: calculated 501.1424, found 501.1424.

Example 8 (Not Inventive)

**3-oxo-5-(propoxycarbonyl)-3H-spiro[isobenzofuran-1,9'-xanthene]-3',6'-diyl diacetate (5-PFDAE)**

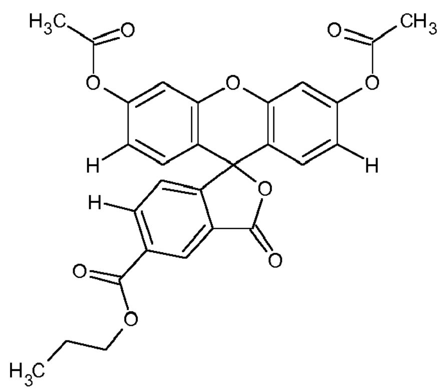

Acetylation of the compound of Example 4 resulted in a yellow solid;Yellow solid; yield 4.2 mg (70%); $t_R$ = 6.28 min, RP-HPLC purity: ≥95%, HR-MS for C28H2209: calculated 502.1264, found 502.1261.

Example 9 (Not Inventive)

**3',6'-diacetoxy-2',7'-difluoro-3-oxo-3H-spiro[isobenzofuran-1,9'-xanthene]-5-carboxylic acid (5-COGDA)**

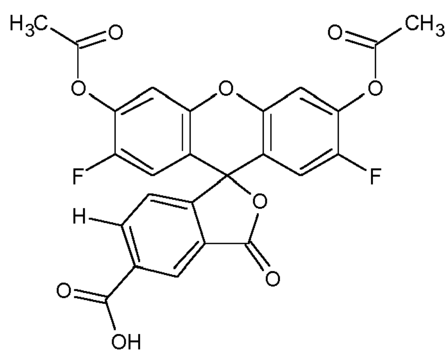

Acetylation of Oregon Green™ 488 carboxylic acid resulted in a yellow solid; yield 10 mg (83%); $t_R$ = 5.10 min, RP-HPLC purity: ≥98%, HR-MS for C25H14F2O9: calculated 496.0606, found 496.0623.

Example 10

**2',7'-difluoro-3-oxo-5-(propylcarbamoyl)-3H-spiroisobenzofuran-1,9'-xanthene-3',6'-diyl diacetoxymethyl ether (5-POGAM)**

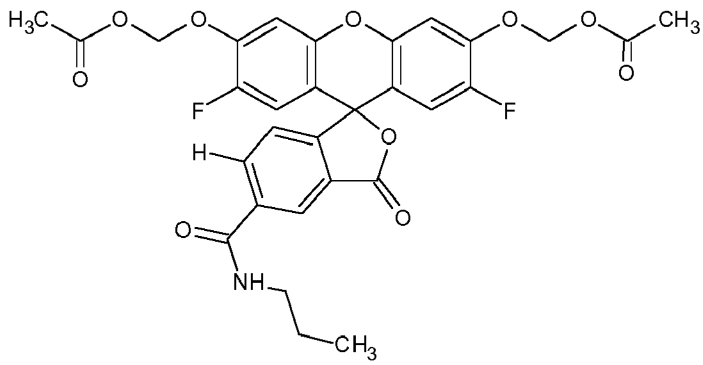

The compound of Example 2, (8.8 mg, 0.019 mmol), powdered 4-A molecular sieves (10 mg), and $Ag_2O$ (11 mg, 0.048 mmol) were suspended in anhydrous $CH_3CN$ (0.5 ml) under argon. Bromomethyl acetate (7.6 µl, 0.077 mmol) was added, and the reaction mixture was stirred for 48h. The reaction mixture was then filtered through a pad of celite. The solution was concentrated under reduced pressure. A purification by reverse-phase HPLC, followed by column chromatography (silica gel, 5 to 20% EtOAc in $CH_2Cl_2$) gives the desired product as a white solid (4.8 mg, 41%). $t_R$ = 5.33 min, RP-HPLC purity: >95%, HR-MS for $C_{31}H_{21}F_2NO_{13}$: calculated 653.0981, found 653.0979.

**Spectroscopic properties**

Absorption spectra were recorded in a 1-cm path length cuvette (Hellma, 104.002B-QS) on a SPECORD®-205 spectrometer from Analytik Jena.

The Extinction coefficients of the flourophores fluorescein, 5-carboxyfluorescein (5-CF), 5-carboxy Oregon Green™ (5-COG), 5-amido N-propyl Oregon Green (5-POG; Example 2) and 5-amido N-propyl fluorescein (5-PFA; Example 3) were measured in 0.1 M NaOH solution at pH 12.7.

The extinction coefficients of the pro-fluorophores 5-thiourea N-propyl fluorescein diacetate (5-PFDAT; Example 5), 5-propyl carboxylate fluorescein diacetate (5-PFDAE; Example 8), 5-amido N-propyl fluorescein diacetate (5-PFDA; Example 7), 5-carboxy Oregon Green diacetate (5-COGDA; Example 9), 5-amido N-propyl Oregon Green diacetate (5-POGDA; Example 6) and 5-amido N-propyl Oregon Green acetoxymethylester (5-POGAM; Example 10) were measured in PBS buffer containing 0.6 % glutaraldehyde at pH 7.

Fluorometric measurements were made using fluorescence grade quartz cuvette and FluoroLog®-3 spectrofluorometer from Horiba Jobin Yvon equipped with sample stirring. The quantum yields (QY) of 5-CF, 5-COG, 5-POG and 5-PFA were measured with samples at 1 µM (absorbance $_{488\ nm}$ ≤ 0.2) in 0.1 M NaOH at pH 12.7. Every sample and the standard (fluorescein) was prepared freshly before use. In this study, fluorescein in 0.1 M NaOH at pH 12.7 was the reference system with a known quantum yield (Φ = 0.925).

Quantum yield measurements were done using standard absorption and emission spectrometers. Emission spectra range was between the wavelengths 498 - 600 nm and excitation wavelength was 488 nm for the samples and fluorescein. The refractive indices of the samples and fluorescein solutions were the same since they were prepared in the same buffer. Under these conditions, quantum yields were calculated by using the equation:

$$\Phi^i = (F^i f_s / F^s f_i)^* \Phi^s$$

wherein $\Phi^i$ and $\Phi^s$ are the photoluminescence QY of the sample and standard (fluorescein), respectively; $F^i$ and $F^s$ are the integrated intensities (areas) of sample and fluorescein spectra, respectively; and $f_i$ and $f_s$ are the absorption

factor of the sample and fluorescein, respectively, with $f_{i\ or\ s} = 1\text{-}10^{-A_{i\ or\ s}}$, wherein A = absorbance.

The same experiment was repeated three times (n = 3).

The spectroscopic properties obtained from these measurements are listed in the following Table 1. The results show that the fluorophores of the present invention present fluorescence characteristics similar to fluorescein, carboxyfluorescein, and carboxy Oregon Green. 5-POG is the fluorophore obtained after unblocking of 5-POGDA or 5-POGAM by ester hydrolysis. The quantum yield of 5-POG ist not significantly altered as compared to fluorescein, carboxyfluorescein and carboxy Oregon Green.

Table 1

| Compound | $\lambda_{Abs\ max}$ (nm) | E ($M^{-1}.cm^{-1}$) | $\lambda_{ex}$ (nm) | $\lambda_{em}$ (nm) | $\Phi$ |
|---|---|---|---|---|---|
| Fluorescein | 491 | 87458 ± 8271 | 490 ± 0.3 | 512 ± 0.5 | 0.925 |
| 5-CF | 493 | 76474 ± 4009 | 491 ± 0.7 | 517 ± 0.6 | 0.963 ± 0.004 |
| 5-COG | 493 | 83136 ± 4452 | 491 ± 1.2 | 517 ± 0.4 | 0,912 ± 0.011 |
| 5-POG | 495 | 74835 ± 4762 | 493 ± 0.6 | 518 ± 1.1 | 0,902 ± 0.013 |
| 5-PFA | 495 | 73490 ± 8454 | 493 ± 0.3 | 518 ± 0.4 | 0,835 ± 0.002 |
| 5-PFDAT | ND | ND | ND | ND | ND |
| 5-PFDAE | 295 | 7903 ± 761 | ND | ND | ND |
| 5-PFDA | 291 | 9284 ± 758 | ND | ND | ND |
| 5-COGDA | 300 | 6979 ± 282 | ND | ND | ND |
| 5-POGDA | 303 | 9784 ± 351 | ND | ND | ND |
| 5-POGAM | 302 | 11165 ± 334 | ND | ND | ND |
| ND: not determined | | | | | |

**Chemical stability tests**

Pro-fluorophores must be stable in aqueous solution to be useful in biological assays. Hydrolysable substrates can have insufficient stability because spontaneous hydrolysis can compete with enzymatic activity and therefore raise the background fluorescence level.

The short-term chemical stability of the pro-fluorophores of the present invention in an aqueous buffer was evaluated by fluorometric measurement analysis after several minutes of storage at 37°C.

Each pro-fluorophore (5-CFDA, 5-PFDAE, 5-PFDAT, 5-PFDA, 5-COGDA, 5-POGDA, 5-POGAM) was dissolved in dimethylsulfoxide (DMSO) in order to prepare a 10 mM stock solution. Short-term stability studies (0-60 min) were performed in the absence of light at 37°C. The pro-fluorophore stock solutions were diluted into a PBS solution containing 0.6 % of glutaraldehyde (pH ~7). The pro-fuorophore final concentration was 15 μM. Samples were stored in a fluorescence grade quartz cuvette having a volume of 1 ml. Fluorescence emission spectra between 500 and 540 nm were recorded every 10 minutes. The excitation wavelength used was 488 nm. Fluorometric measurement were made with a FluoroLog®-3. (Horiba Jobin Yvon) spectrofluorometer equipped with sample stirring. The same experiment was repeated three times (n = 3).

As shown in Figure 1, the straight line represents the fluorescence intensity of the formed product during time. Thus, the slope of the straight line corresponds to the speed of spontaneous fluorescence generation. The relative speed of spontaneous fluorescence generation from 5-POGAM is almost four times slower than that of 5-CFDA. 5-PFDAE showed also remarkable stability in PBS solution containing 0.6 % glutaraldehyde. In contrast, 5-COGDA and 5-POGDA have rapid spontaneous hydrolysis in this solution, eight times higher and almost six times higher, respectively, than 5-CFDA.

The above tests indicate that pro-fluorophores with fluorine substitution, like 5-COGDA and 5-POGDA, are less stable as compared to 5-CFDA and 5-PFDA having no fluorine substitution. The data confirm that fluorine substituted pro-fluorophores are more prone to hydrolysis in an aqueous buffer solution.

To examine whether the pro-fluorophores 5-POGDA and 5-POGAM could be stored for at least two months in solution at room temperature (23 ± 2 °C) and in the absence of light, they were dissolved in anhydrous DMSO solvent and analysed by liquid chromatography-mass spectrometry. The analysis was performed using an UHPLC-MS/MS system (LC-MS 8030, Shimadzu). 5-POGDA or AM (120 μM) were stored in anhydrous DMSO. A solution of 5-CFDA

(20 μM) in acetonitrile stored at -80°C was used as internal reference for HPLC-MS/MS analysis. The solution injected was composed of 5-POGDA or AM (1.2 μM), and 5-CFDA (0,2 μM) in $H_2O$/acetonitrile, ratio 1/1 (V/V).

Data acquisition and analysis were performed using LabSolutions version 5 software. A 1.7 μm Kinetex™ column (50 X 2.1 mm) was used for HPLC. The mobile phase flow rate was fixed at 0.5 ml/min and the following program was applied for the elution: 0-1.2 min, 5-95% B; 1.2-1.4 min, 95% B; 1.4-1.42 min, 95-0% B and 1.42-2.8 min, 5% B. Solvent A consisted of 0.05% formic acid in water and solvent B was HPLC grade acetonitrile. 1 μl was injected.

The mass spectrometer was interfaced with the UHPLC system using an electrospray ion source. The instrumental parameters were as follows: nebulizer gas, 2 l /min; DL temperature, 250°C; block heater temperature, 400°C; interface voltage, 4.5 kV; collision energy, between -12 and -22V. The collision gas used was argon at 230 kPa. The MRM transitions were m/z 598.2 → 454.1, 538.1 → 454.1 and 454.1 → 368.0, respectively for 5-POGAM, 5-POGDA and 5-POG. The dwell time was set to 30 msec and the pause time to 3 msec. The percentage of remaining test compound relative to $t_0$ was measured using the peak area on the chromatogram.

The result of the long-term stability tests are shown in Fig. 2. The percentage of 5-POGDA and 5-POGAM remaining in the solution is calculated to be around 18 % and 65 % respectively after 77 days. The side product of 5-POGAM in DMSO after 77 days found by mass scan corresponded to mono-hydrolysed 5-POGAM. Conversely, in the solution of 5-POGDA, the majority amount was the fluorophore 5-POG shown by mass scan analysis, and no mono-hydrolysed product was detected. The present results suggest that for a long-term storage (< 2 months) of the pro-fluorophore in solution at room temperature in darkness, 5-POGAM can be dissolved in anhydrous DMSO in a disposable system.

Together, the stability tests show that 5-POGAM (Example 10) possess greater chemical stability in aqueous solution.

**Bacteria Staining Tests**

Esterases are present in all living organisms, and these enzymes can be used to provide information on bacterial viability. The pro-fluorophores of the present invention, 5-POGDA, 5-POGAM, and a prior art compound, 5-CFDA were compared with respect to their ability to stain several bacterial species.

*Escherichia coli (E. coli)* (ATCC 8739) and *Bacillus subtilis (B. subtilis)* (ATCC 6633) strains were routinely grown in Luria Bertani (LB) broth or on LB agar plates for 18h at 37 °C. Waterborne environmental bacteria such as *Enterobacter cloacae (E. cloacae), Chryseobacterium indologenes (C. indologenes), Pseudomonas aeruginosa (P. aeruginosa),* and E. *coli (EC18)* were purchased from Eurofins™. Environmental bacteria were grown in LB broth or on casein-peptone soymeal-peptone agar plates for 18h at 37 °C.

For the staining tests, *E. coli* (ATCC 8739 or EC18), *E. cloacae, C. indologenes, P. aeruginosa* and *B. subtilis* were cultivated in Luria-Bertani (LB) broth until exponential phase at 37°C with shaking at 300 rpm revolutions per minutes (rpm). Bacteria were harvested and washed in sterile phosphate buffer saline solution (PBS) at pH -7.4 by centrifugation at 10000 rpm for 3 min at room temperature (21 ± 2 °C). Then the bacteria were resuspended in PBS at a concentration of $1 \cdot 10^9$ bacteria/ml. Bacteria concentration was calculated according to the turbidity measure at 660 nm ($A_{660nm}$=1 correspond to bacterial suspension at ~ $5 \cdot 10^8$ bacteria/ ml).

Dead bacterial suspensions at $1 \cdot 10^9$ bacteria/ml (negative controls) of these five species of bacteria were prepared individually by addition of 20 μl of bleach containing 2.6 % active chlorine. Bacteria were treated with bleach solution for 15 min. Dead bacteria were washed twice in sterile PBS by centrifugation at 10000 rpm for 3 min at room temperature. The culturability of the negative controls was assessed by plating 100 μl of bacterial suspension onto LB agar or Caso agar and incubating the plates at 37 °C for 72 h. None of the negative controls displayed any growth activity.

Bacteria (100 μl suspension at $1 \cdot 10^9$ bacteria/ml) were stained with 10 μM or 15 μM of pro-fluorophore (5-CFDA, 5-PFDAE, 5-PFDAT, 5-PFDA, 5-COGDA, 5-POGDA or 5-POGAM) in 200 μl of PBS solution containing 0.6 % of glutaraldehyde. Bacteria were incubated at 37 °C for 10 - 60 min with pro-fluorophore in darkness. After bacterial staining, they were washed and resuspended in 100 μl of PBS as described above.

Staining of the bacterial suspension was observed under a fluorescent microscope. These bacteria were imaged with a Leica DM5500 microscope equipped with FITC filter. Brightfield images indicated that bacteria appeared to have normal physiology. Images were collected with a 100x objective. Fluorescence intensity of stained bacterial suspension was also measured with FluoroLog®-3 (Horiba Jobin Yvon) spectrofluorometer equipped with sample stirring. Samples were placed in fluorescence grade quartz cuvette having a volume of 1 ml. The excitation wavelength used was 488 nm and emission wavelength used was 517 nm. The same experiment was repeated three times (n = 3).

As shown in Figure 3, treatment of bacterial suspensions with 5-POGDA and 5-POGAM stained only viable *E. cloacae* (Fig. 3a), *C. indologenes* (Fig. 3b), *and P. aeruginosa* (Fig. 3c), which are all gram-negative bacteria, and also viable (gram-positive) *B. subtilis* (Fig. 3d). No fluorescence signal was detected by microscopy analysis with the dead bacteria. The same results were obtained with other species such as the coliform bacteria *Citobacter freudii, Klebsiella oxytoca* and the non-coliform bacteria *Chryseobacterium indologenes* and *Comamonas testosteroni*/*alcaligenes* (data

not shown).

Moreover, viable gram-negative bacteria like *E. Cloacae, C. indologenes, P. aeruginosa* incubated with 5-POGDA or 5-POGAM have brighter fluorescence than 5-CFDA stained bacteria. As shown in Figure 3d, the fluorescence intensity of the stained *B. subtilis* with 5-CFDA is higher than the staining with 5-POGDA and 5-POGAM. These results suggest that the pro-fluorophores 5-POGDA and 5-POGAM are specifically useful for detecting viable gram-negative bacteria.

**Labeling of viable *E. coli***

The pro-fluorophores according to the present invention are preferably used to label E. *coli* cells. Viable or dead *E. coli* (ATCC 8739) are incubated with these fluorophores and commercial 5-CFDA as described above. Fluorescent microscopy analysis is used to show that 5-PFDAE, 5-PFDAT, 5-PFDA, 5-COGDA, 5-POGDA and 5-POGAM can be unblocked by the bacterial endogenous esterase activity. Glutaraldehyde is added to the incubation solutions to improve the retention of the fluorophores in bacteria.

Figure 4 shows microphotographs of viable or dead *E. coli* (ATCC 8739) incubated with different pro-fluorophores (15 μM) for 30 min at 37 °C, overlaid with brightfield images. Bacteria were washed before being imaged with a Leica DM5500 microscope, 100x objective. Scale bars indicate 10 μm.

As shown in Figure 4, viable *E. coli* can be specifically stained with high fluorescence intensity using the esterified pro-fluorophores 5-PFDA, 5-COGDA, 5-POGDA and 5-POGAM.

Viable E. coli treated with 5-PFDAE showed a weaker green fluorescence, as compared to 5-CFDA staining. No fluorescence signal of dead bacteria was detected. However, dead and viable *E. coli* treated with 5-PFDAT become fluorescent. Thus, 5-PFDAT treatment is not adapted to distinguish between viable or dead *E. coli.* It is assumed that the thiourea functional group of 5-PFDAT interacts with compounds of the *E. coli* membrane. Dead *E. coli* fluorescence staining with 5-PFDAT was probably due to non-enzymatic hydrolysis.

Figure 5 shows the effect of the pro-fluorophore on the fluorescence intensity. Viable or dead *E. coli* (ATCC 8739) were incubated with different pro-fluorophores (10 - 15 μM) for 30 min at 37 °C. After washes, fluorescence of the *E. coli* suspension was measured with a FluoroLog®-3 spectrofluorometer (excitation wavelength: 488 nm, emission wavelength: 517 nm). Relative rates of viable *E. coli* fluorescence intensity were calculated by normalizing the difference of viable *E. coli* fluorescence intensity and dead *E. coli* fluorescence intensity with 5-CFDA staining.

The Bacterial fluorescence intensity measured by spectrofluorometry shows that there is no fluorescence intensity gain with 5-PFDAE containing an ester functional group, as compared to 5-CFDA which contains carboxylic acid functional group.

However, as also observed in the microscopy analysis, viable *E. coli* cells incubated for 30 min at 37 °C with 5-POGDA exhibit a fluorescence intensity approximately 6 times higher than 5-CFDA treated viable *E. coli.*

Viable *E. coli* stained with 5-POGAM, 5-PFDAE or with 5-COGDA, have a fluorescence intensity between two and three times higher than with 5-CFDA staining.

Further experiments show that an increase of viable *E. coli* fluorescence intensity is obtained up to about 15 μM for 5-POGDA and 5-POGAM. Using higher concentrations of μM of 5-POGDA or 5-POGAM in the range between 20 - 40 μM resulted in a saturation process. 40 μM of 5-POGDA or 5-POGAM still provide a stronger *E. coli* staining than 5-CFDA or 5-PFDA (data not shown).

Figure 6 shows the effect of the incubation time on viable *E. coli* fluorescence intensity. Viable *E. coli* (ATCC 8739) were incubated with different pro-fluorophores (15 μM) at 37 °C for 10 - 60 min. After washes, *E. coli* fluorescence suspension was measured with FluoroLog®-3 spectrofluorometer (excitation wavelength: 488 nm, emission wavelength: 517 nm).

As can be seen in Figure 5, longer pro-fluorophore incubation with E. coli results in an increase of fluorescence intensity accumulated in the bacteria. The differences of the fluorescence signals between the respective pro-fluorophores are conserved throughout 60 minutes. These results suggest that tested pro-fluorophores differ with respect to uptake in the bacteria and hydrolysis speed of the blocking group. The addition of an amido propyl functional group (5-PFDA) instead of a carboxylic functional group (5-CFDA) promotes the pro-fluorophore uptake into *E. coli,* since 5-PFDA incubation for 10 to 60 min shows a higher fluorescence intensity than incubation with 5-CFDA.

Fluorine substitution of 5-PFDA gives 5-POGDA having a higher hydrolysis rate, as shown in Figure 1. Accordingly, 5-POGAM offers a compromise between greater chemical stability and a slower but still efficient unblocking reaction by esterase hydrolysis in *E. coli* as compared to 5-POGDA.

An incubation time of 30 min at 37 °C was found to be sufficient for generating a strong fluorescence signal in order to easily distinguish viable from dead *E. coli,* and at the same time providing a low background due to spontaneous hydrolysis of the pro-fluorophore. Thus, incubation with 5-POGAM (15 μM, 37 °C) has the advantage of producing a three times brighter *E. coli* fluorescence than 5-CFDA and being much more stable in aqueous solution, as compared to 5-PFDA and 5-COGDA.

**Immunofluorescence and viability staining of *E. coli***

One of the currently challenge in the water industry is to detect specific viable bacteria in drinking water. Actually, the need for more rapid and sensitive tests is essential. The above tests show that viable laboratory strains of *E. coli* are advantageously stained with pro-fluorophores of the present invention such as 5-POGAM and 5-POGDA.

In the following tests, different waterborne *E. coli* species are detected using an immunological approach.

Environmental *E. Coli* (EC18) were cultivated and re-suspended in PBS as described above. A dead bacterial suspension was also prepared as previously described. Dead or viable *E. coli* (EC18) (100 μl of suspension at $1.10^9$ bacteria/ml) were incubated with rabbit anti-EC18 polyclonal antibodies (anti-EC18 pAb) (dilution 1/20) for 1 h at room temperature. This anti-EC 18 pAb was obtained from immunized rabbit with inactivated EC18 (Eurogentec). Bacteria were washed in PBS by centrifugation at 10000 rpm for 3 min at room temperature. Then, they were resuspended in 200 μl of PBS solution containing 0.6 % of glutaraldehyde with 15 μM of 5-POGDA and incubated for 30 min at 37 °C in the absence of light. Following several washes with PBS as described above, dead or viable *E. coli* (EC18) were re-suspended in 200 μl of PBS and incubated for 1 h at room temperature in the absence of light with AlexaFluor 568 labeled anti-rabbit immunoglobulin (dilution 1/1000) (Molecularprobes, A11036). Bacteria were washed twice and re-suspended in 100 μl of PBS. They were imaged with a Leica DM5500 microscope equipped with FITC and Cy3 filters. Images were collected with a 100x objective. The same experiment was repeated three times (n = 3).

Dead and viable *E. coli* (EC18) were visualized as red cells after incubation with rabbit anti-EC18 polyclonal antibodies (anti-EC18 pAb) and AlexaFluor 568 labeled anti-rabbit immunoglobulin. Staining of viable *E. coli* with 5-POGDA resulted in a bright green fluorescence. Bacteria were washed before imaged with a Leica DM5500 microscope, 100x objective. Scale bars: 10 μm (data not shown).

The immunofluorescence assay indicated that the anti-EC18 pAb reacts equally with viable and dead cells. When these treated cells coated with antibodies are incubated with 5-POGDA, viable cells have a green fluorescence and dead cells have no green fluorescence.

In a further test, antibodies anti-EC18 pAB and pro-fluorophores (5-POGDA, 5-POGAM) were used to immobilize *E. coli* (EC18) on beads and to reveal the immobilized bacteria respectively. Immunocapture of *E. coli* on beads was done in order to concentrate the fluorescent signal in order to improve the detection of the viable cells. In these experiments, Protein G Sepharose beads coated with goat-anti-rabbit immunoglobulins were used.

Environmental *E. coli* (E18) were cultivated and re-suspended in PBS as previously described. *E. coli* (EC18) (100 μl of suspension at $1{*}10^9$ bacteria/ml) were incubated with rabbit anti-EC18 polyclonal antibodies (anti-EC18 pAb) or pre-immune control rabbit serum without anti-EC18 antibodies (PI-pAb) (dilution 1/20) for 1 h at room temperature. This control rabbit serum (PI-pAb) was obtained from a non-immunized animal (Eurogentec). It was used as a negative control. Bacteria were washed three times in PBS by centrifugation at 10000 rpm for 3 min at room temperature.

Proteine G Sepharose 4 Fast Flow beads were washed three times in Milli-Q water and three times in PBS by centrifugation at 2000 rpm for 2 min at 4 °C. Then 10 μl of sedimented beads were incubated with 20 μg of secondary antibody goat-anti-rabbit immunoglobulin (Thermo Scientific Pierce, 31210) in 500 μl of PBS with 0.001% of NP40 for 1h at room temperature with gentle mixing on a wheel. After two washes with PBS, these 10 μl of sedimented Protein G Sepharose beads coated with goat-anti-rabbit immunoglobulins were incubated with *E. coli* (100 μl of suspension at $1.10^9$ bacteria/ml) coated with rabbit anti-EC18 antibodies in 400 μl of PBS with 0.001% of NP40 for 2 h at room temperature with gentle mixing on a wheel. After several washes, bacteria immobilized on sedimented beads were stained with 10 μM 5-CFDA or 5-POGDA or 5-POGAM in 200 μl of PBS and 0.6 % glutaraldehyde for 30 min at 37 °C in darkness. Beads were washed twice and 50 μl of Trypan Blue solution (0.4 %) was added as a contrast agent. Beads were imaged with a Leica DM5500 microscope equipped with FITC filters. Images were collected with a 63x objective.

Observation by fluorescence microscopy showed a green fluorescent signal corresponding to viable *E. coli* (EC18) captured on beads. However, no fluorescent signal was detected on the beads in absence of anti-EC18 antibodies (PI-pAb), suggesting that *E. coli* (EC18) cells did not bind unspecifically to the beads. Moreover, fluorescence intensity of viable *E. coli* stained with 5-POGDA or 5-POGAM was still higher than the fluorescence intensity of *E. coli* stained with 5-CFDA.

These results suggest that uptake of 5-POGDA and 5-POGAM is not hindered by the presence of antibodies at the bacterial surface. Moreover, 5-POGDA or 5-POGAM used to stain viable bacteria did not perturb the binding between antibodies and bacteria. Therefore, 5-POGAM and 5-POGDA are useful for the detection of immobilized bacteria.

Other useful contrast agents are Commassie™ Brilliant Blue R and Bromophenol Blue. Briliant Blue R has properties comparable to Trypan Blue but can be considered as a non CMR vital contrast agent. It is able to absorb the fluorescence emitted from the fluorophores of the present invention, in particular 5-POG, but is exluded from vital cells. Therefore, Brilliant Blue R is preferably used to eliminate all background due to non-enzymatic ester hydrolysis. Bromophenol Blue will penetrate living cells and erase all fluorescence. Use of Bromophenol Blue therefore allows for calibrating the fluorescence signal avoiding background after washes.

## Claims

1. A compound represented by the following formula (I)

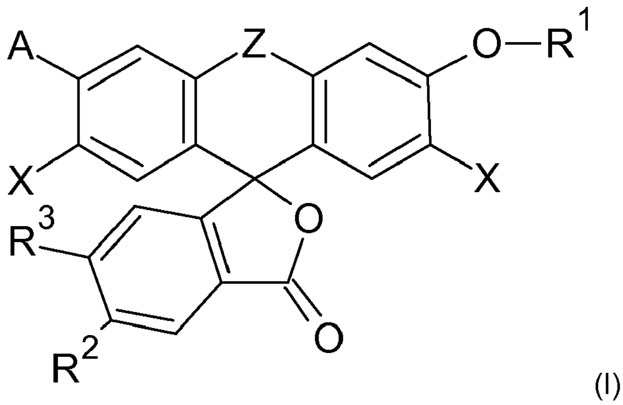

(I)

wherein
each X is, independently from each other, one of H, F, Cl, Br, I, CN;
Z is one of O, Si, Ge or Sn;
$R^1$ is hydrogen, $-(C{=}O)-R^4$ or $-CH_2-O-(C{=}O)-R^4$, wherein $R^4$ is substituted or unsubstituted alkyl having 1 to 18 carbon atoms, substituted or unsubstituted alkenyl having 2 to 18 carbon atoms, or substituted or unsubstituted aralkyl having at least 7 carbon atoms, or wherein $R^1$ is a residue derived from an amino acid, peptide, nucleotide or oligonucleotide, monosaccharide or polysaccharide;
A is $-NH_2$, $-OR^1$ or $-NR^5(C{=}O)-R^4$ wherein $R^1$ and $R^4$ independently have the meaning as defined above and $R^5$ is H, substituted or unsubstituted alkyl having 1 to 18 carbon atoms, substituted or unsubstituted alkenyl having 2 to 18 carbon atoms, or substituted or unsubstituted aralkyl having at least 7 carbon atoms;
$R^2$ and $R^3$ are, independently from each other, H or $-(C{=}O)-NR^6R^7$, wherein at least one of $R^2$ and $R^3$ is not H, and wherein $R^6$ and $R^7$ are selected from H, substituted or unsubstituted alkyl having 1 to 18 carbon atoms, substituted or unsubstituted alkenyl having 2 to 18 carbon atoms, or substituted or unsubstituted aralkyl having at least 7 carbon atoms.

2. The compound of claim 1, represented by the following formula (II):

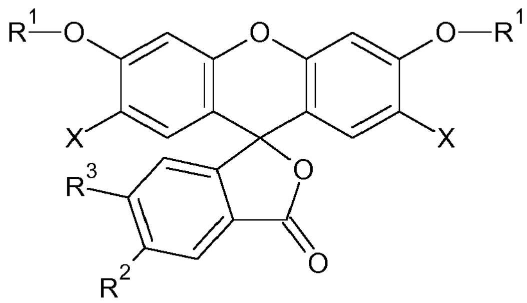

(II)

wherein X, $R^2$ and $R^3$ each have the meaning as defined above, each $R^1$ are the same or different from each other and are hydrogen, $-(C{=}O)-R^4$ or $-CH_2-O-(C{=}O)-R^4$, wherein $R^4$ independently is substituted or unsubstituted alkyl having 1 to 18 carbon atoms, substituted or unsubstituted alkenyl having 2 to 18 carbon atoms, or substituted or unsubstituted aralkyl having at least 7 carbon atoms.

3. The compound of claim 2, wherein X is H or F, and $R^4$ independently is alkyl having 1 to 8 carbon atoms, more preferably methyl or ethyl.

4. The compound of claim 2 or 3, wherein $R^2$ or $R^3$ is $-(C{=}O)-NH-R^7$, wherein $R^7$ is alkyl having 1 to 8 carbon atoms, more preferably alkyl having 1 to 4 carbon atoms.

**5.** The compound of any one of claims 2 to 4, wherein X is H, both $R^1$ are either -(C=O)-$R^4$ or -$CH_2$-O-(C=O)-$R^4$, $R^4$ is methyl, and one of $R^2$ and $R^3$ is -(C=O)-NH-$R^7$, wherein $R^7$ is selected from methyl, ethy, propyl, isopropy, butyl, and isobutyl.

**6.** The compound of any one of claims 2 to 4, wherein X is F, both $R^1$ are either -(C=O)-$R^4$ or -$CH_2$-O-(C=O)-$R^4$, $R^4$ is methyl, and one of $R^2$ and $R^3$ is -(C=O)-NH-$R^7$ wherein $R^7$ is selected from methyl, ethy, propyl, isopropy, butyl and isobutyl.

**7.** The compound of claim 1, represented by the following formula (III):

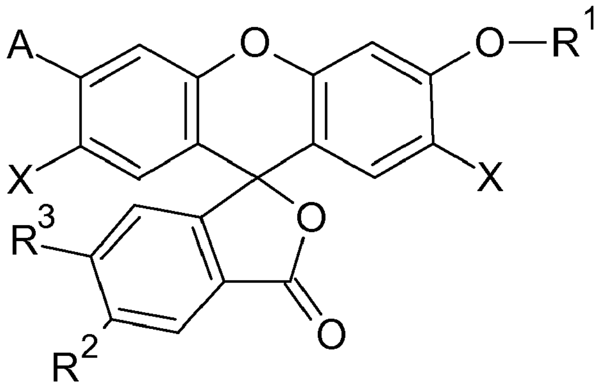

(III)

wherein X, $R^2$ and $R^3$ have the meaning as defined above, A is -$NH_2$, or-$NR^5$(C=O)-$R^4$, $R^1$ is hydrogen, -(C=O)-$R^4$ or -$CH_2$-O-(C=O)-$R^4$, wherein each $R^4$ independently is substituted or unsubstituted alkyl having 1 to 18 carbon atoms, substituted or unsubstituted alkenyl having 2 to 18 carbon atoms, or substituted or unsubstituted aralkyl having at least 7 carbon atoms, and wherein $R^5$ is H, substituted or unsubstituted alkyl having 1 to 18 carbon atoms, substituted or unsubstituted alkenyl having 2 to 18 carbon atoms, or substituted or unsubstituted aralkyl having at least 7 carbon atoms.

**8.** The compound of claim 7, wherein X is H or F, $R^4$ independently is alkyl having 1 to 4 carbon atoms, more preferably methyl or ethyl.

**9.** The compound of claim 8, wherein $R^5$ is H or alkyl having 1 to 4 carbon atoms, more preferably H, methyl or ethyl.

**10.** The compound according to any one of claims 7 to 9, wherein $R^2$ or $R^3$ is -(C=O)-NH-$R^7$, wherein $R^7$ is alkyl having 1 to 8 carbon atoms, preferably alkyl having 1 to 4 carbon atoms.

**11.** The compound of claim 7, wherein X is H, A is -$NR^5$(C=O)-$R^4$, $R^1$ is either -(C=O)-$R^4$ or -$CH_2$-O-(C=O)-$R^4$, $R^4$ is methyl, $R^5$ is H, and one of $R^2$ and $R^3$ is - (C=O)-NH-$R^8$ wherein $R^8$ is selected from methyl, ethy, propyl, isopropy, butyl, isobutyl.

**12.** The compound of claim 7, wherein X is F, A is -$NR^5$(C=O)-$R^4$, $R^1$ is either -(C=O)-$R^4$ or -$CH_2$-O-(C=O)-$R^4$, $R^4$ is methyl, $R^5$ is H, and one of $R^2$ and $R^3$ is - (C=O)-NH-$R^8$ wherein $R^8$ is selected from methyl, ethy, propyl, isopropy, butyl, isobutyl.

**13.** Use of a compound according to any one of claims 1 to 12 for staining a biological sample.

**14.** Use of claim 13 wherein the biological sample comprises a living cell.

**15.** Use of claim 14 wherein the sample cell is incubated with the compound of any one of claims 1 to 12 for a time sufficient for the compound to yield a detectable fluorescence emission.

**16.** Use of claim 14 wherein the fluorescence emission is evaluated to discriminate living from non-living cells.

**17.** Use of any one of claims 14 to 16 wherein the living cell is a unicellular and multicellular organism.

18. Use of any one of claims 12 to 17 wherein the biological sample is subjected to a cytometric assay.

19. Use of any one of claims 13 to 18 wherein the biological sample is treated with a contrast agent.

20. Use of claim 19 wherein the contrast agent is selected from the group consisting of Brilliant Blue R, Trypan Blue and Bromophenol Blue.

21. Culture medium for microorganisms, comprising a nutrient medium and a compound according to any one of claims 1 to 12 dissolved in the nutrient medium.

# Fig. 1

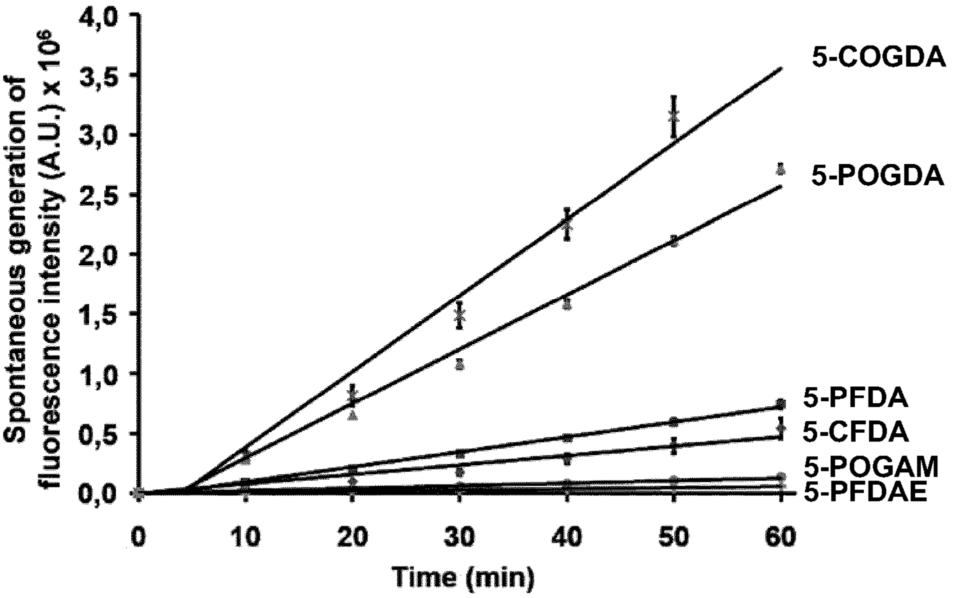
Spontaneous generation of fluorescence intensity (A.U.) x 10^6
4,0
3,5
3,0
2,5
2,0
1,5
1,0
0,5
0,0
0
10
20
30
40
50
60
Time (min)
5-COGDA
5-POGDA
5-PFDA
5-CFDA
5-POGAM
5-PFDAE

# Fig. 2

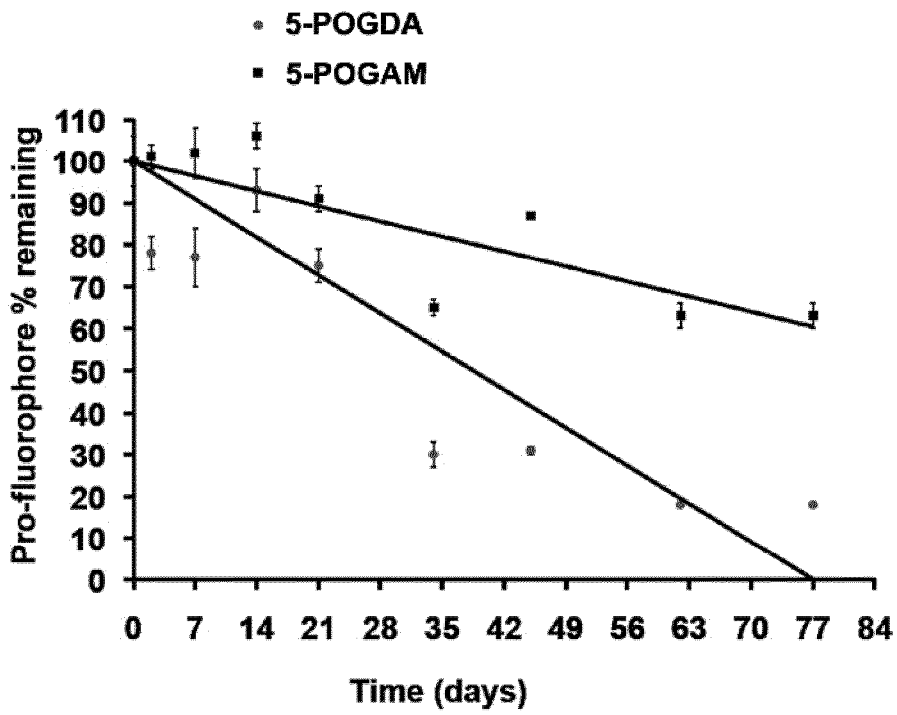
5-POGDA
5-POGAM
Pro-fluorophore % remaining
110
100
90
80
70
60
50
40
30
20
10
0
0
7
14
21
28
35
42
49
56
63
70
77
84
Time (days)

# Fig. 3

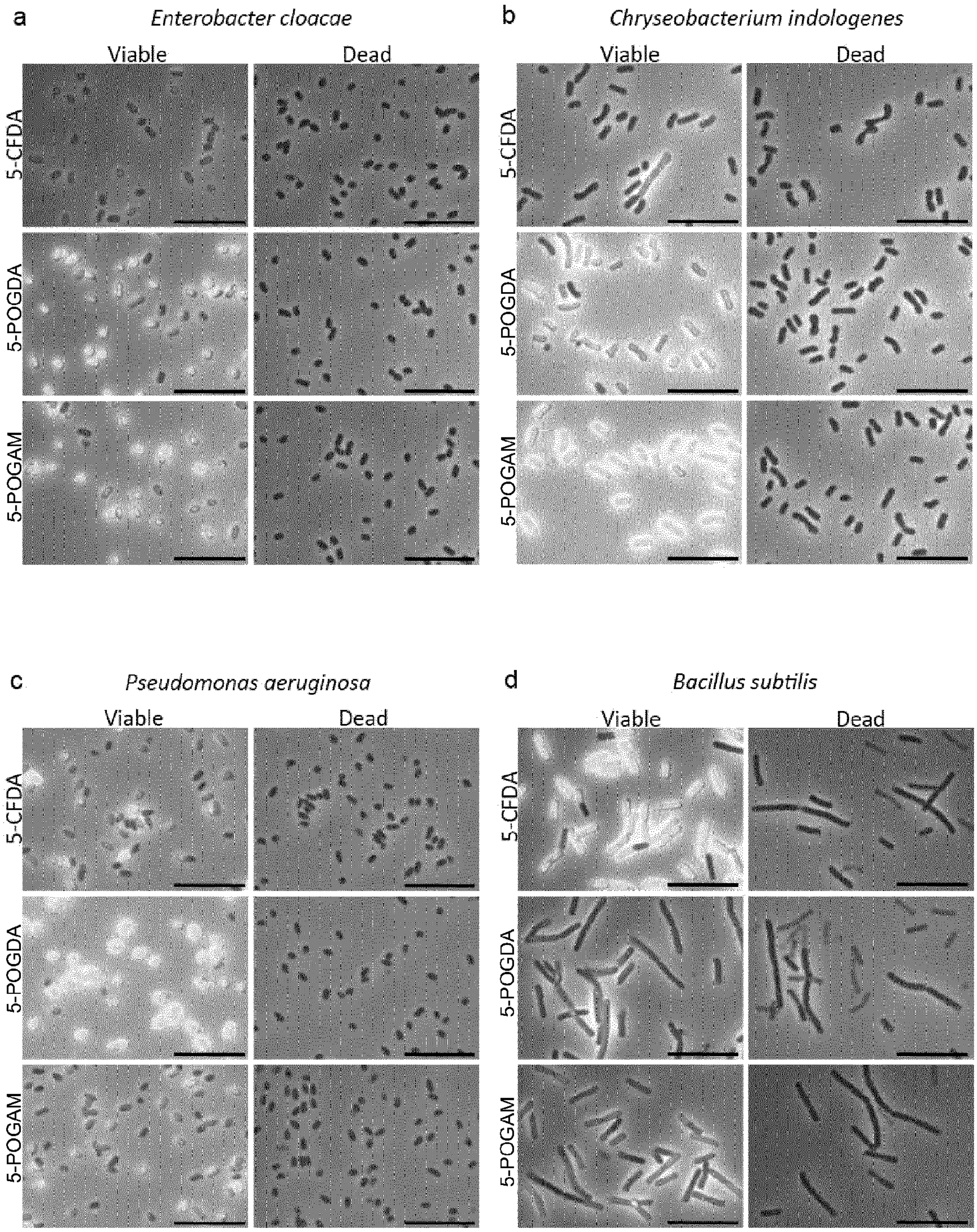
a
Enterobacter cloacae
Viable
Dead
5-CFDA
5-POGDA
5-POGAM
b
Chryseobacterium indologenes
Viable
Dead
5-CFDA
5-POGDA
5-POGAM
c
Pseudomonas aeruginosa
Viable
Dead
5-CFDA
5-POGDA
5-POGAM
d
Bacillus subtilis
Viable
Dead
5-CFDA
5-POGDA
5-POGAM

Fig. 4

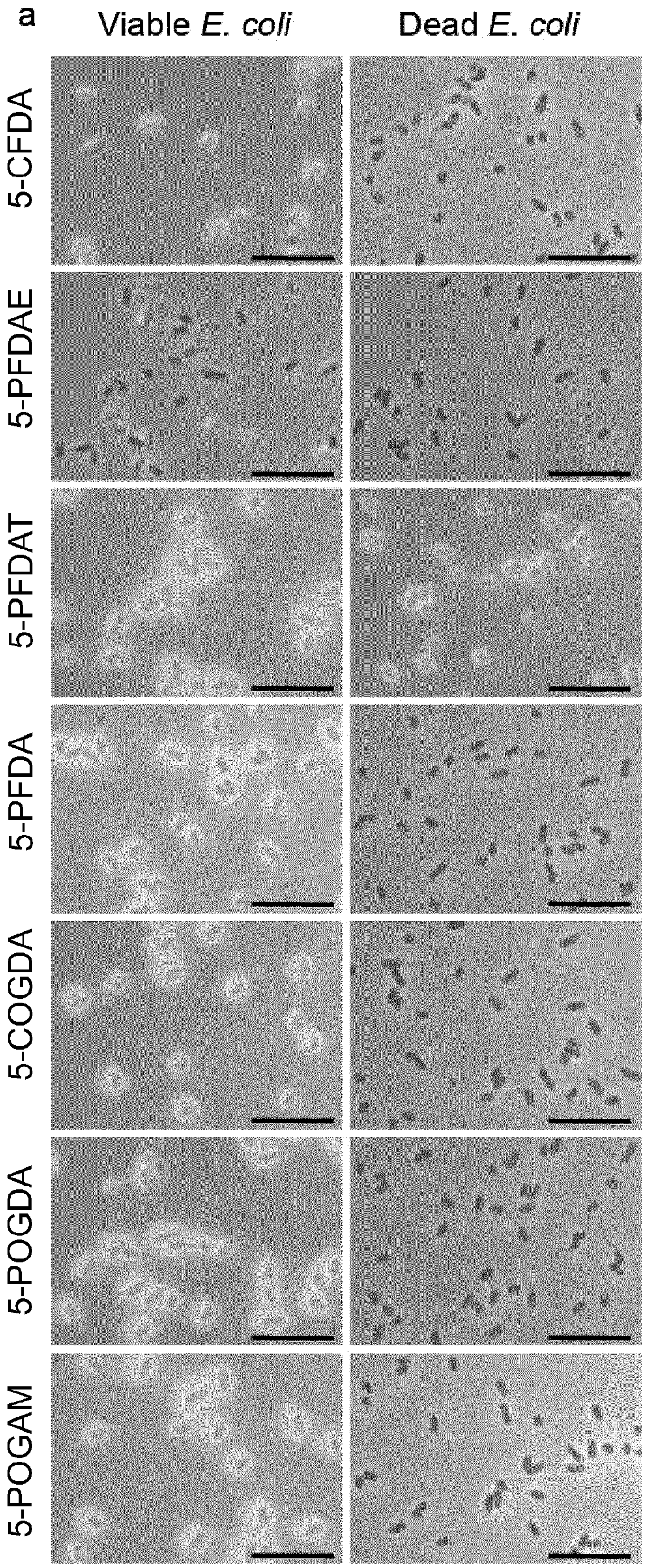
a
Viable E. coli
Dead E. coli
5-CFDA
5-PFDAE
5-PFDAT
5-PFDA
5-COGDA
5-POGDA
5-POGAM

Fig. 5

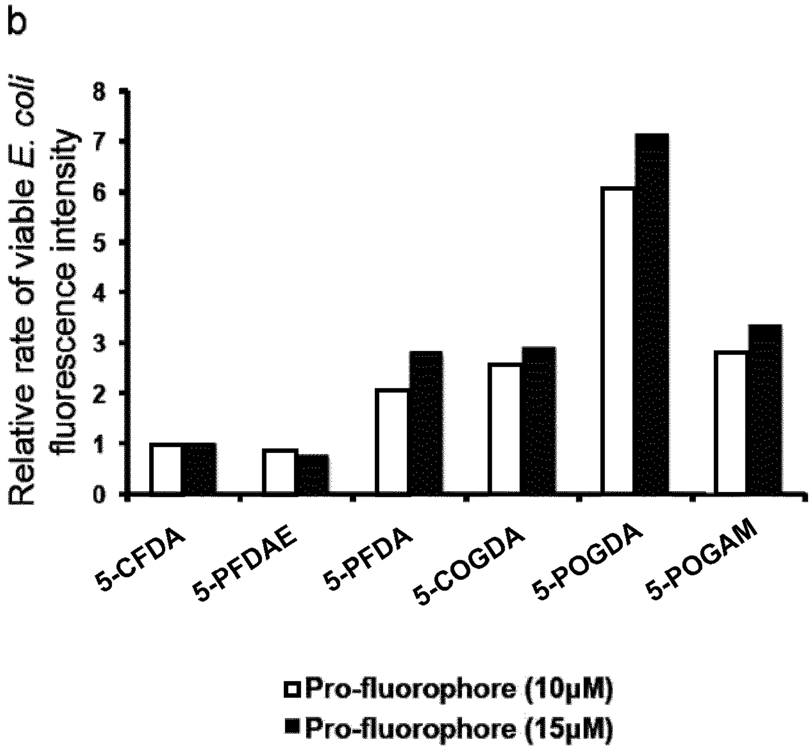
b
Relative rate of viable E. coli fluorescence intensity
0
1
2
3
4
5
6
7
8
5-CFDA
5-PFDAE
5-PFDA
5-COGDA
5-POGDA
5-POGAM
Pro-fluorophore (10µM)
Pro-fluorophore (15µM)

Fig. 6

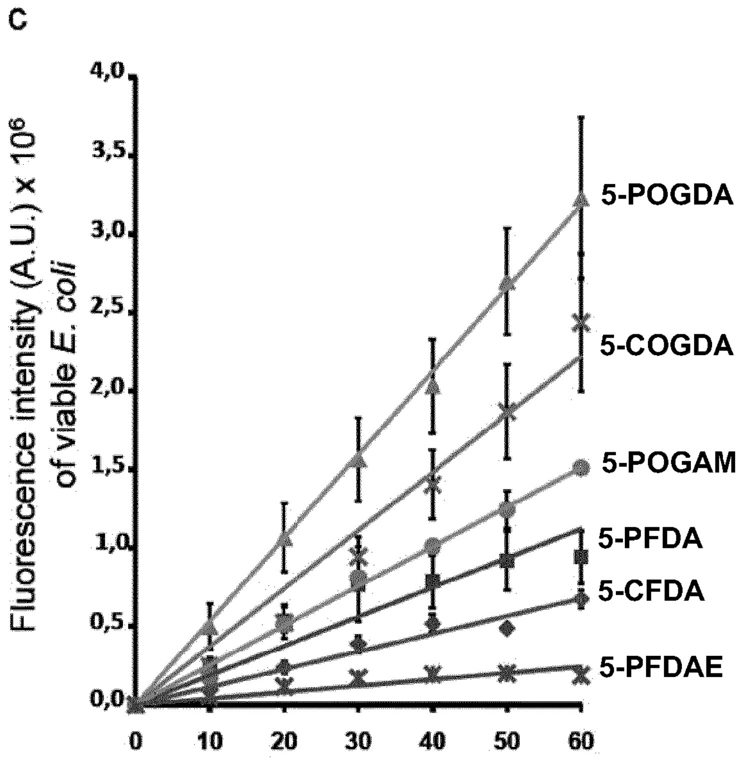
c
Fluorescence intensity (A.U.) x $10^6$ of viable E. coli
0,0
0,5
1,0
1,5
2,0
2,5
3,0
3,5
4,0
0
10
20
30
40
50
60
5-POGDA
5-COGDA
5-POGAM
5-PFDA
5-CFDA
5-PFDAE

Europäisches Patentamt
European Patent Office
Office européen des brevets

# EUROPEAN SEARCH REPORT

Application Number
EP 15 15 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/128898 A1 (ENZO LIFE SCIENCES INC [US]; COLEMAN JACK [US]; RABBANI ELAZAR [US]; S) 27 September 2012 (2012-09-27)<br>* page 22; claim 34 * | 1-3, 13-15 | INV.<br>C07D493/10<br>A61K31/35<br>A61K31/365<br>C09B11/06<br>C09K11/06<br>C09K11/59<br>C09K11/66<br>C12N1/00<br>C12Q1/02<br>G01N33/48<br>G01N33/50<br>G01N21/64<br>G01N33/52 |
| X | WO 2011/073137 A2 (BAYER SCHERING PHARMA AG [DE]; MUELLER ANDRE [DE]; ZITZMANN-KOLBE SABI)<br>23 June 2011 (2011-06-23)<br>* claim 20; examples 4,5,12,14,15,17 * | 1-3, 13-17 | |
| X | WO 2010/129929 A1 (UNIV ILLINOIS [US]; MILLER LARRY [US])<br>11 November 2010 (2010-11-11)<br>* scheme 10, compound 2b; scheme 12, compound 3b * | 1-3, 13-15 | |
| X | WO 2004/011900 A2 (ACLARA BIOSCIENCES INC [US]) 5 February 2004 (2004-02-05)<br>* figures 5B, 6A-J, 7A-C * | 1-3, 13-15 | TECHNICAL FIELDS SEARCHED (IPC) |
| X | WO 02/095356 A2 (ACLARA BIOSCIENCES INC [US]; SINGH SHARAT [US]; SALIMI-MOOSAVI HOSSEIN) 28 November 2002 (2002-11-28)<br>* figures 1, 2, 4, 15A-J, 16A, 16F-I * | 1-3,13 | C07D<br>A61K<br>C09B<br>C09K<br>C12N<br>C12Q<br>G01N |
| X | WO 01/83502 A1 (ACLARA BIOSCIENCES INC [US]) 8 November 2001 (2001-11-08)<br>* schemes 1, 2, 3; tables 1, 2, 5 * | 1-3, 13-15 | |
| X | WO 00/66607 A1 (ACLARA BIOSCIENCES INC [US]) 9 November 2000 (2000-11-09)<br>* schemes 1, 2, 3; tables 1, 2, 4 * | 1-3, 13-15 | |

-/--

The present search report has been drawn up for all claims

2

| Place of search | Date of completion of the search | Examiner |
|---|---|---|
| Berlin | 23 March 2015 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 97/39064 A1 (MOLECULAR PROBES INC [US]) 23 October 1997 (1997-10-23)<br>* pages 17-18; examples 72, 74, 77, 79 *<br>----- | 1-3, 13-17 | |
| X | US 2008/171351 A1 (SMITH BRADLEY D [US]) 17 July 2008 (2008-07-17)<br>* claims 7, 26; figure 7B *<br>----- | 1-3, 13-17 | |
| X,D | HOEFEL, DANIEL ET AL: "A comparative study of carboxyfluorescein diacetate and carboxyfluorescein diacetate succinimidyl ester as indicators of bacterial activity",<br>JOURNAL OF MICROBIOLOGICAL METHODS,<br>vol. 52, no. 3, 2003, pages 379-388,<br>XP002589397,<br>DOI: 10.1016/S0167-7012(02)00207-5<br>* abstract; figure 1 *<br>----- | 1-3, 13-17 | |
| X | KELLER, MICHAEL ET AL: "Nuclear localisation sequence templated nonviral gene delivery vectors: Investigation of intracellular trafficking events of LMD and LD vector systems",<br>CHEMBIOCHEM,<br>vol. 4, no. 4, 2003, pages 286-298,<br>XP002737542,<br>DOI: 10.1002/CBIC.200390049<br>* scheme 1, compound 6 *<br>-----<br>-/-- | 1-3, 13-17 | TECHNICAL FIELDS SEARCHED (IPC) |

The present search report has been drawn up for all claims

2

| Place of search | Date of completion of the search | Examiner |
|---|---|---|
| Berlin | 23 March 2015 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIANG, PENGJU ET AL: "Fluorescent detection of zinc in biological systems: recent development on the design of chemosensors and biosensors", COORDINATION CHEMISTRY REVIEWS, vol. 248, no. 1-2, 2004, pages 205-229, XP002737543, DOI: 10.1016/J.CCT.2003.10.013 * scheme 21, compound 88 * ----- | 1-3, 13-17 | |
| X | YUEN, CHUN-TING ET AL: "A fluorescent peptide substrate for measuring the ADP-ribosylation activity of the cholera toxin A-subunit", HUMAN VACCINES, vol. 2, no. 5, 2006, pages 195-199, XP002737544, DOI: 10.4161/HV.2.5.3105 * page 196; figure 1 * ----- | 1-3,13 | |
| | | | TECHNICAL FIELDS SEARCHED (IPC) |
| X | LEE, YA-JUNG ET AL: "Real-Time Fluorescence Detection of Protein Transduction into Live Cells", JOURNAL OF THE AMERICAN CHEMICAL SOCIETY, vol. 130, no. 8, February 2000 (2000-02), pages 2398-2399, XP002737545, DOI: 10.1021/JA7102026 * figure 1 * ----- | 1-3,13, 14 | |
| X | QUE, EMILY L. ET AL: "Metals in Neurobiology: Probing Their Chemistry and Biology with Molecular Imaging", CHEMICAL REVIEWS, vol. 108, no. 5, 2008, pages 1517-1549, XP002737546, DOI: 10.1021/CR078203U * chart 5, compound 49 * ----- -/-- | 1-3 | |

2

The present search report has been drawn up for all claims

| Place of search | Date of completion of the search | Examiner |
|---|---|---|
| Berlin | 23 March 2015 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DUAN, XINRUI ET AL: "Homogeneous and one-step fluorescent allele-specific PCR for SNP genotyping assays using conjugated polyelectrolytes", BIOSENSORS & BIOELECTRONICS, vol. 24, no. 7, 2009, pages 2095-2099, XP002737547, DOI: 10.1016/J.BIOS.2008.10.027 * scheme 1(b),, compounds dGTP-FI, dUTP-FI * ----- | 1-3,13 | |
| X | GARANGER, ELISABETH ET AL: "Divergent Oriented Synthesis For the Design of Reagents for Protein Conjugation", JOURNAL OF COMBINATORIAL CHEMISTRY, vol. 12, no. 1, 2010, pages 57-64, XP002737548, DOI: 10.1021/CC900141B * figure 3, FI derivatives * ----- | 1-3,13 | |
| X | KLIPPEL, STEFAN ET AL: "Cell tracking with Caged Xenon: Using Cryptophanes as MRI Reporters upon Cellular Internalization", ANGEWANDTE CHEMIE, INTERNATIONAL EDITION, vol. 53, no. 2, 2014, pages 493-496, XP002737549, DOI: 10.1002/ANIE.201307290 * figure 2; compound CrA-FAM * ----- | 1-3, 13-15 | TECHNICAL FIELDS SEARCHED (IPC) |
| A | EP 1 256 624 A1 (MATSUSHITA SEIKO KK [JP] MATSUSHITA ECOLOGY SYS CO [JP]) 13 November 2002 (2002-11-13) * abstract; claims * ----- | 1-21 | |
| | -/-- | | |

The present search report has been drawn up for all claims

| Place of search | Date of completion of the search | Examiner |
|---|---|---|
| Berlin | 23 March 2015 | Frelon, Didier |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

European Patent Office / Europäisches Patentamt / Office européen des brevets

# EUROPEAN SEARCH REPORT

Application Number
EP 15 15 3463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LLOYD, QIN P. ET AL: "Characterization of calcium translocation across the plasma membrane of primary osteoblasts using a lipophilic calcium-sensitive fluorescent dye, Calcium Green C18", JOURNAL OF BIOLOGICAL CHEMISTRY, vol. 270, no. 38, 1995, pages 22445-22451, XP002737550, DOI: 10.1074/JBC.270.38.22445 * page 22448; figure 1 * ----- | 1-21 | |
| A | SHANER, NATHAN C. ET AL: "Improved monomeric red, orange and yellow fluorescent proteins derived from Discosoma sp. red fluorescent protein", NATURE BIOTECHNOLOGY, vol. 22, no. 12, 2004, pages 1567-1572, XP002737551, DOI: 10.1038/NBT1037 * abstract; table 1 * ----- | 1-21 | |
| A | SHORTREED, MICHAEL ET AL: "Fluorescent Fiber-Optic Calcium Sensor for Physiological Measurements", ANALYTICAL CHEMISTRY, vol. 68, no. 8, 1996, pages 1414-1418, XP002737552, DOI: 10.1021/AC950944K * abstract; figure 2 * ----- | 1-21 | TECHNICAL FIELDS SEARCHED (IPC) |
| A | THOMPSON, R. B. ET AL: "Fluorescent zinc indicators for neurobiology", JOURNAL OF NEUROSCIENCE METHODS, vol. 118, no. 1, 2002, pages 63-75, XP002737553, DOI: 10.1016/S0165-0270(02)00144-9 * figure 2 * ----- | 1-21 | |

The present search report has been drawn up for all claims

2

| Place of search | Date of completion of the search | Examiner |
|---|---|---|
| Berlin | 23 March 2015 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 15 15 3463

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 15 15 3463

The Search Division considers that the present European patent application does not comply with the requirements of unity of invention and relates to several inventions or groups of inventions, namely:

1. claims: 1-21

Compounds of formula (I)

1.1. claims: 1, 7-21(all partially)

(group of) invention(s) (I) when A represents an amino substituent, possibly acylated as NR5 CO-R4

1.2. claims: 2-6(completely); 1, 7-21(partially)

(group of) invention(s) (II) when A represents a OR1 group

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO. EP 15 15 3463

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2015

| Patent document cited in search report | Publication date | Patent family member(s) | Publication date |
|---|---|---|---|
| WO 2012128898 A1 | 27-09-2012 | CA 2827754 A1 | 27-09-2013 |
| | | EP 2686684 A1 | 22-01-2014 |
| | | US 2011318788 A1 | 29-12-2011 |
| | | WO 2012128898 A1 | 27-09-2012 |
| WO 2011073137 A2 | 23-06-2011 | TW 201136609 A | 01-11-2011 |
| | | WO 2011073137 A2 | 23-06-2011 |
| WO 2010129929 A1 | 11-11-2010 | US 2012115128 A1 | 10-05-2012 |
| | | WO 2010129929 A1 | 11-11-2010 |
| WO 2004011900 A2 | 05-02-2004 | AT 442589 T | 15-09-2009 |
| | | AU 2003259175 A1 | 16-02-2004 |
| | | CA 2490654 A1 | 05-02-2004 |
| | | CN 1672054 A | 21-09-2005 |
| | | EP 1540347 A2 | 15-06-2005 |
| | | ES 2332310 T3 | 02-02-2010 |
| | | JP 4443407 B2 | 31-03-2010 |
| | | JP 2005534030 A | 10-11-2005 |
| | | JP 2009271087 A | 19-11-2009 |
| | | KR 20050025669 A | 14-03-2005 |
| | | US 2004126818 A1 | 01-07-2004 |
| | | US 2004197835 A1 | 07-10-2004 |
| | | WO 2004011900 A2 | 05-02-2004 |
| WO 02095356 A2 | 28-11-2002 | CA 2445053 A1 | 28-11-2002 |
| | | CN 1559006 A | 29-12-2004 |
| | | CZ 20033143 A3 | 14-04-2004 |
| | | EP 1506399 A2 | 16-02-2005 |
| | | JP 2005505749 A | 24-02-2005 |
| | | KR 20040082273 A | 24-09-2004 |
| | | NO 20035010 A | 11-11-2003 |
| | | US 2003013126 A1 | 16-01-2003 |
| | | US 2008311674 A1 | 18-12-2008 |
| | | US 2012088307 A1 | 12-04-2012 |
| | | WO 02095356 A2 | 28-11-2002 |
| WO 0183502 A1 | 08-11-2001 | AT 398135 T | 15-07-2008 |
| | | AU 1240201 A | 12-11-2001 |
| | | AU 2001212402 B2 | 04-11-2004 |
| | | CA 2403326 A1 | 08-11-2001 |
| | | DK 1278760 T3 | 13-10-2008 |
| | | EP 1278760 A1 | 29-01-2003 |
| | | ES 2308999 T3 | 16-12-2008 |
| | | HU 0300371 A2 | 28-06-2003 |
| | | JP 3566255 B2 | 15-09-2004 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 15 15 3463

This annex lists the patent family members relating to the patent documents cited in the above-mentioned European search report. The members are as contained in the European Patent Office EDP file on
The European Patent Office is in no way liable for these particulars which are merely given for the purpose of information.

23-03-2015

| Patent document cited in search report | Publication date | Patent family member(s) | Publication date |
|---|---|---|---|
| | | JP 2003532092 A | 28-10-2003 |
| | | JP 2004117376 A | 15-04-2004 |
| | | KR 20030032939 A | 26-04-2003 |
| | | PL 364923 A1 | 27-12-2004 |
| | | US 6514700 B1 | 04-02-2003 |
| | | US 2003175747 A1 | 18-09-2003 |
| | | US 2004029139 A1 | 12-02-2004 |
| | | WO 0183502 A1 | 08-11-2001 |
| WO 0066607 A1 | 09-11-2000 | AU 763671 B2 | 31-07-2003 |
| | | AU 4497200 A | 17-11-2000 |
| | | AU 2003252906 A1 | 06-11-2003 |
| | | CA 2368581 A1 | 09-11-2000 |
| | | EP 1180112 A1 | 20-02-2002 |
| | | JP 2002542811 A | 17-12-2002 |
| | | US 6322980 B1 | 27-11-2001 |
| | | US 2001049105 A1 | 06-12-2001 |
| | | US 2002001808 A1 | 03-01-2002 |
| | | US 2002009737 A1 | 24-01-2002 |
| | | US 2002015954 A1 | 07-02-2002 |
| | | US 2002045738 A1 | 18-04-2002 |
| | | US 2002058263 A1 | 16-05-2002 |
| | | US 2002090616 A1 | 11-07-2002 |
| | | US 2004166529 A1 | 26-08-2004 |
| | | US 2004265858 A1 | 30-12-2004 |
| | | WO 0066607 A1 | 09-11-2000 |
| WO 9739064 A1 | 23-10-1997 | AT 279480 T | 15-10-2004 |
| | | AU 717569 B2 | 30-03-2000 |
| | | AU 2801297 A | 07-11-1997 |
| | | CA 2222275 A1 | 23-10-1997 |
| | | DE 69731179 D1 | 18-11-2004 |
| | | DE 69731179 T2 | 13-10-2005 |
| | | EP 0853647 A1 | 22-07-1998 |
| | | EP 1441010 A1 | 28-07-2004 |
| | | JP 4408451 B2 | 03-02-2010 |
| | | JP H11508277 A | 21-07-1999 |
| | | US 6162931 A | 19-12-2000 |
| | | US 6229055 B1 | 08-05-2001 |
| | | WO 9739064 A1 | 23-10-1997 |
| US 2008171351 A1 | 17-07-2008 | US 2008171351 A1 | 17-07-2008 |
| | | US 2010331542 A1 | 30-12-2010 |
| EP 1256624 A1 | 13-11-2002 | AU 2711201 A | 14-08-2001 |
| | | AU 2001227112 B2 | 15-06-2006 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 15 15 3463

This annex lists the patent family members relating to the patent documents cited in the above-mentioned European search report. The members are as contained in the European Patent Office EDP file on
The European Patent Office is in no way liable for these particulars which are merely given for the purpose of information.

23-03-2015

| Patent document cited in search report | Publication date | Patent family member(s) | Publication date |
|---|---|---|---|
| | | AU 2006202405 A1 | 29-06-2006 |
| | | CA 2399508 A1 | 09-08-2001 |
| | | DE 60130970 T2 | 31-07-2008 |
| | | EP 1256624 A1 | 13-11-2002 |
| | | EP 1679364 A1 | 12-07-2006 |
| | | EP 1854873 A2 | 14-11-2007 |
| | | US 2003003527 A1 | 02-01-2003 |
| | | US 2008220439 A1 | 11-09-2008 |
| | | WO 0157180 A1 | 09-08-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4318846 A
- WO 1997039064 A1
- FR 2955121 A
- WO 2006003696 A1
- JP 2004187534 A
- US 2008220439 A1


### Non-patent literature cited in the description


- **YUICHIRO KOIDE et al.** Evolution of group 14 rhodamines as platforms for near-infrared fluorescence probes utilizing photoinduced electron transfer. *ACS Chem. Biol.,* 2011, vol. 6, 600-608
- **T.H. CHRZANOWSKI et al.** Applicability of the fluorescein diacetate method of detecting active bacteria in fresh water. *Microb Ecol,* 1984, vol. 10, 179-185
- **THOMAS A. ROBERTSON et al.** Fluorescein Derivatives in Intravital Fluorescence Imaging. *Cells,* 2013, vol. 2 (3), 591-606
- **L. D. LAVIS et al.** Synthesis and utility of fluorogenic acetoxymethyl ethers. *Chem Sci.,* 2011, vol. 2, 521-530
- **D. HOEFEL et al.** A comparative study of carboxyfluorescein diacetate and carboxyfluorescein diacetate succinimidyl ester as indicators of bacterial activity. *Journal of Microbiological Methods,* 2003, vol. 52, 379-388
- **P. BREEUWER et al.** Characterization of uptake and hydrolysis of fluorescein diacetate and carboxyfluorescein diacetate by intracellular esterases in Saccharomyces cerevisiae, which result in accumulation of fluorescent product. *Appl Environ Microbiol.,* April 1995, vol. 61 (4), 1614-1619
- **VINCE BOYD et al.** Limitations in the Use of Fluorescein Diacetate/Propidium Iodide (FDA/PI) and Cell Permeable Nucleic Acid Stains for Viability Measurements of Isolated Islets of Langerhans. *Curr Trends Biotechnol Pharm .,* March 2008, vol. 2 (2), 66-84